(19) Europäisches Patentamt
European Patent Office
Office européen des brevets

(11) **EP 4 104 756 A1**

(12) **EUROPÄISCHE PATENTANMELDUNG**

(43) Veröffentlichungstag:
**21.12.2022 Patentblatt 2022/51**

(21) Anmeldenummer: **21179522.4**

(22) Anmeldetag: **15.06.2021**

(51) Internationale Patentklassifikation (IPC):
**A61B 5/11** (2006.01)    **A61B 5/113** (2006.01)
**A61B 5/00** (2006.01)    **A61B 5/0245** (2006.01)
**A61B 5/318** (2021.01)    **A61B 5/389** (2021.01)

(52) Gemeinsame Patentklassifikation (CPC):
**A61B 5/113; A61B 5/0245; A61B 5/1126;**
**A61B 5/318; A61B 5/389; A61B 5/7207;**
**A61B 5/7267; A61B 5/7292;** A61B 5/277;
A61B 5/6892; A61B 2562/0214

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder: **Batzer, Ulrich**
**91080 Spardorf (DE)**

(54) **DIFFERENTIELLES SPANNUNGSMESSSYSTEM ZUR MESSUNG DER ATMUNGSAKTIVITÄT EINES PATIENTEN**

(57) Ein differentielles Spannungsmesssystem zur Bestimmung einer Atmungsaktivität eines Patienten weist eine Anzahl von Signalmessschaltungen mit jeweils einem kapazitiven Sensorelement zur Erfassung eines auf den Patienten bezogenen Messsignals auf. Das differentielle Spannungsmesssystem weist ferner eine Signalverarbeitungsvorrichtung zur Bestimmung wenigstens eines bioelektrischen Signals aus den Messignalen und eine Recheneinheit auf, die dazu ausgebildet ist, basierend auf dem wenigstens einen bioelektrischen Signal eine Atmungsinformation zu ermitteln und diese bereitzustellen, welche Atmungsinformation eine Atmungsaktivität des Patienten indiziert.

FIG 2

Processed by Luminess, 75001 PARIS (FR)

EP 4 104 756 A1

**Beschreibung**

Die Erfindung betrifft ein differentielles Spannungsmesssystem zur Messung einer Atmungsaktivität eines Patienten. Außerdem betrifft die Erfindung ein Verfahren zur Bestimmung einer Atmungsaktivität eines Patienten mit einem differentiellen Spannungsmesssystem.

Für bildgebende Verfahren ist es nötig, dass sich der aufgenommene Abschnitt in Ruhe befindet. Bei Aufnahmen im Bereich des Thorax, inklusive kardiologischer Aufnahmen, ist neben der Bewegung des Herzens vor allem die Bewegung durch Atmung störend.

Für die Messung einer Herzaktivität haben sich Spannungsmesssysteme etabliert, die zur Messung von bioelektrischen Signalen ausgebildet sind. Diese werden in der Medizin standardmäßig zur Messung von Elektrokardiogrammen (EKG), Elektroenzephalogrammen (EEG) oder Elektromyogrammen (EMG) genutzt. Hierfür können Sensoren verwendet werden, die am Körper des Patienten befestigt werden müssen. Alternativen hierzu stellen sog. kapazitives Messsysteme dar, bei denen ein EKG-Signal rein kapazitiv abgegriffen wird, ohne direkten Kontakt des Patienten zum Sensor zu haben, insbesondere durch die Kleidung des Patienten hindurch. Für den Patienten ist dies besonders komfortabel, da die kapazitive EKG-Messung ohne Anlegen oder Befestigen von einzelnen Sensoren erfolgen kann. Hierfür ist bekannt, die Sensorik in die Oberfläche einer Patientenliege, bspw. einer Bildgebungsanlage oder in eine Auflage-Matte oder in eine (Sitz-) Lehne zu integrieren, sodass die Spannungsmessung erfolgen kann, sobald der Patient auf der Patientenliege oder dem Sitz positioniert ist. Eine derartige Vorrichtung ist bspw. in der deutschen Patentanmeldung DE 10 2015 218 298 B3 beschrieben. Die so gewonnen Informationen über die Herzaktivität können einer angeschlossenen Bildgebungsanlage zugeführt werden, um die Bildgebung an die Herzaktivität anzupassen und störende Effekte durch den Herzschlag des Patienten bei der Bildgebung zu eliminieren oder wenigstens zu berücksichtigen.

Um durch eine Atmungsaktivität des Patienten erzeugte störende Effekte auf die Bildgebung zu reduzieren, besteht immerhin die Möglichkeit, den Patienten die Luft anhalten zu lassen. Dies ist allerdings nicht allen Patienten möglich, wird oft falsch ausgeführt und führt mitunter zu starken Veränderungen des Herzschlags, was die Synchronisation auf diesen erschwert.

Es wäre folglich wünschenswert, die Atmungsaktivität des Patienten wie bei der Messung der Herzaktivität messtechnisch zu erfassen und bei der Bildgebung zu berücksichtigen. Hierfür wären aus dem Stand der Technik Messsysteme wie Atemmessgurte oder Kamera- oder Radar-basierten Atmungserkennungssysteme bekannt. Solche Systeme haben allerdings den Nachteil, dass zusätzliche Komponenten bereitgestellt und implementiert werden müssen. Zudem hat dies zur Folge, dass bei der Bildgebung zwei Signale unterschiedlicher Messsysteme, dem System zur Messung einer Herzaktivität und dem Atmungserkennungssystem, berücksichtigt und letztlich in irgendeiner Form synchronisiert werden müssen, was die Abstimmung der Bildgebung auf die Messsignale erschwert.

Es ist eine Aufgabe der vorliegenden Erfindung, hier Abhilfe zu schaffen und ein Messsystem und zugehörige Verfahren bereitzustellen, die eine verbesserte Messung einer Atmungsaktivität eines Patienten ermöglichen und insbesondere gut mit der Messung einer Herzaktivität des Patienten kompatibel sind.

Diese Aufgabe wird gelöst durch ein differentielles Spannungsmesssystem zur Messung einer Atmungsaktivität eines Patienten bzw. ein Verfahren zur Messung einer Atmungsaktivität eines Patienten gemäß den unabhängigen Ansprüchen. Bevorzugte und/oder alternative, vorteilhafte Ausgestaltungen sind Gegenstand der abhängigen Ansprüche und der nachfolgenden Beschreibung. Nachstehend wird die erfindungsgemäße Lösung der Aufgabe sowohl in Bezug auf die beanspruchten Systeme als auch in Bezug auf die beanspruchten Verfahren beschrieben. Hierbei erwähnte Merkmale, Vorteile oder alternative Ausführungsformen/Aspekte sind ebenso auch auf die anderen beanspruchten Gegenstände zu übertragen und umgekehrt. Mit anderen Worten können die gegenständlichen Ansprüche (die beispielsweise auf ein System gerichtet sind) auch mit den Merkmalen, die in Zusammenhang mit einem Verfahren beschrieben oder beansprucht sind, weitergebildet sein und umgekehrt. Die entsprechenden funktionalen Merkmale des Verfahrens können dabei durch entsprechende gegenständliche Module ausgebildet sein.

Weiterhin wird die erfindungsgemäße Lösung der Aufgabe auch in Bezug auf Verfahren zum Anpassen von trainierten Funktionen beschrieben. Hierbei können Merkmale und alternative Ausführungsformen/Aspekte von Datenstrukturen und/oder Funktionen bei Verfahren und Vorrichtungen zur Bestimmung einer Atmungsaktivität auf analoge Datenstrukturen und/oder Funktionen bei Verfahren und Vorrichtungen zum Anpassen einer trainierten Funktion übertragen werden. Analoge Datenstrukturen können hierbei insbesondere durch die Verwendung der Vorsilbe "Trainings-" gekennzeichnet sein. Weiterhin können die in Verfahren und Systemen zur Bestimmung einer Atmungsaktivität eines Patienten verwendeten trainierten Funktionen insbesondere durch Verfahren und Vorrichtungen zum Anpassen von trainierten Funktionen angepasst worden und/oder bereitgestellt worden sein.

Gemäß einem Aspekt wird ein differentielles Spannungsmesssystem zur Bestimmung einer Atmungsaktivität eines Patienten bereitgestellt. Das Spannungsmesssystem weist auf: eine Anzahl von Signalmessschaltungen mit jeweils einem kapazitiven Sensorelement zur Erfassung eines auf den Patienten bezogenen Messsignals, eine Signalverarbeitungsvorrichtung zur Bestimmung wenigstens eines bioelektrischen Signals aus den Messignalen; und eine Recheneinheit, die dazu ausgebildet ist, basierend auf dem wenigstens einen bioelektrischen Signal eine Information zu ermitteln und diese bereitzustellen, welche Information eine Atmungsaktivität des Patienten indiziert (im Folgenden auch Atmungsinformation genannt).

Differentielle Spannungsmesssysteme als solche sind dem Fachmann ihrer grundlegenden Funktionsweise nach bekannt, weswegen an dieser Stelle auf eine detailliertere Erläuterung verzichtet wird. Sie können herkömmlich insbesondere zur Bereitstellung von Elektrokardiogrammen (EKG), Elektroenzephalogrammen (EEG) oder Elektromyogrammen (EMG) ausgebildet sein.

Die kapazitiven Sensorelemente können gemäß einigen Ausführungsformen jeweils eine kapazitive Sensorelektrode aufweisen, die galvanisch von dem Patienten getrennt oder mit dem Patienten beispielsweise durch die Kleidung über eine hohe Impedanz, vorzugsweise mit einer Impedanz von mehr als 1 MOhm, elektrisch verbunden ist. Es ist sogar vorteilhaft, die oberste Schicht des kapazitiven Sensorelements leitfähig auszuprägen und damit eine schwache galvanische Verbindung zu schaffen. Ein Vorteil einer schwachen galvanischen Verbindung besteht darin, dass damit eine bessere Signalübertragung für Signalanteile mit tiefen Frequenzen erreicht wird. Ist einer Kapazität ein ohmscher Widerstand parallelgeschaltet, so bildet diese Anordnung für die niedrigen Frequenzen eine niedrigere Impedanz aus, als die Kapazität allein. Außerdem ermöglicht eine solche Verbindung eine Entladung im Rahmen eines ESD-Schutzes. Ferner werden für den Fall, dass ein maximaler ohmscher Widerstand, beispielsweise von 100 MOhm, vorgegeben ist, die Anforderungen an die Eingangsimpedanz der mit dem Sensorelement verbundenen Signalmessschaltung bzw. von deren Eingangsbeschaltung reduziert, da der ohmsche Widerstand der Sensorelektrode mit der Eingangsimpedanz einen Spannungsteiler bildet. Ein solcher Fall liegt vor, wenn die Kleidung des Patienten entsprechend ausreichend elektrisch leitfähig ist, insbesondere nicht aus Wolle besteht.

Die kapazitiven Sensorelemente können jeweils über entsprechende Nutzsignalpfade mit der zugehörigen Signalmessschaltung verbunden sein. Die Nutzsignalpfade können als Sensorleitungen bzw. einzelne Kabel ausgebildet sein. Die Signalmessschaltungen können dabei jeweils als Verstärkerschaltung ausgebildet sein und beispielsweise einen Operationsverstärker umfassen, der insbesondere als Nachläufer ausgebildet sein kann. Die Signalmessschaltungen können jeweils auch als Eingangs-Puffer für die mit den Sensorelementen erfassten Messignale fungieren und als solche bezeichnet werden. Gemäß einigen Ausführungsbeispielen können die Signalmessschaltungen einen AD-Wandler aufweisen, der dazu ausgebildet ist, die durch die Sensorelemente aufgenommenen Messignale zu digitalisieren. Alternativ kann ein AD-Wandler auch in der Signalverarbeitungsvorrichtung ausgebildet oder separat

ausgeführt sein. Die Messignale sind dabei insbesondere elektrische Signale, die durch ein Verhalten oder durch Eigenschaften eines menschlichen oder tierischen Patienten moduliert sind und diese Eigenschaften oder Verhalten somit indizieren bzw. anzeigen. Indizieren bzw. anzeigen kann dabei insbesondere hei-ßen, dass auf diese Eigenschaften oder Verhalten mit Mitteln der Signal- und/oder Datenverarbeitung aus den Messignalen geschlossen werden kann. Eine Eigenschaft bzw. ein Verhalten kann in diesem Fall insbesondere eine Herzaktivität oder eine Atmungsaktivität sein.

Das differentielle Spannungsmesssystem kann insbesondere mindestens ein erstes kapazitives Sensorelement und ein zweites kapazitives Sensorelement zur Messung der Messignale aufweisen. Weiterhin weist das erfindungsgemäße differentielle Spannungsmesssystem vorzugsweise mindestens ein drittes kapazitives Sensorelement mit einem dritten Nutzsignalpfad für einen Potentialausgleich zwischen einem Messobjekt, beispielsweise einem Patienten, und dem differentiellen Spannungsmesssystem auf. Der dritte, vorzugsweise vorgesehene Nutzsignalpfad sorgt für einen Potentialausgleich zwischen dem Patienten und dem kapazitiven differentiellen Spannungsmesssystem. Dabei ist der kapazitive Sensor des dritten Nutzsignalpfads vorzugsweise auf dem rechten Bein des Patienten angebracht, worauf die Bezeichnung "Right-Leg-Drive-Pfad" zurückzuführen ist. Grundsätzlich kann dieses dritte Potential aber auch an anderer Stelle am Patienten erfasst werden.

Die durch die Sensorelemente und die zugehörigen Signalmessschaltungen erfassten Messignale werden in die Signalverarbeitungsvorrichtung eingegeben, die dazu ausgebildet ist, die Messignale zu einem oder mehreren bioelektrischen Signalen weiterzuverarbeiten. Mit anderen Worten stellt ein bioelektrisches Signal also ein auf Grundlage der Messignale erzeugtes Signal dar. Das so erzeugte bioelektrische Signal bzw. die so erzeugten bioelektrischen Signale sind derart ausgestaltet, dass sie eine Atmungsaktivität des Patienten indizieren bzw. durch die Atmungsaktivität des Patienten moduliert sind. Die Signalverarbeitungsvorrichtung kann somit als Vorverarbeitungsstufe aufgefasst werden, die der Ermittlung der Atmungsinformation in der Recheneinheit vorgeschaltet ist. Zur Bestimmung solcher bioelektrischer Signale kann die Signalverarbeitungsvorrichtung beispielsweise dazu ausgebildet sein, geeignete Messignale von entsprechenden Sensorelementen auszuwählen, relevante Messignalanteile (insbesondere relevante Frequenzanteile) zu identifizieren zu extrahieren und/oder zu verstärken, und/oder irrelevante Signalanteile, insbesondere Störanteile, abzudämpfen und/oder zu eliminieren. Zur Bestimmung eines oder mehrerer bioelektrischer Signale kann die Signalverarbeitungsvorrichtung durch Hardware- und/oder Software-Komponenten entsprechend ausgestaltet sein. Beispielsweise kann die Signalverarbeitungsvorrichtung über geeignete Filter- und/oder Verstärkerelemente verfügen und/oder Computerprogrammprodukte zur Ausführung bringen, die diese Elemente virtuell implementieren.

Das oder die bioelektrischen Signale werden in eine Recheneinheit übermittelt, die dazu ausgebildet ist, aus dem oder den bioelektrischen Signalen eine Atmungsinformation des Patienten zu ermitteln. Die Recheneinheit kann dabei z.B. als Teil der Signalverarbeitungsvorrichtung ausgebildet sein. Umgekehrt kann die Signalverarbeitungsvorrichtung als Teil der Recheneinheit ausgebildet sein. Die Recheneinheit kann insbesondere ein Computerprogrammprodukt zur Ausführung bringen, das zur Ermittlung der Atmungsinformation ausgestaltet bzw. angepasst ist. Die Atmungsinformation kann nach einigen Ausführungsbeispielen eine Information über die Ein- und Ausatemvorgänge des Patienten, insbesondere in quasi-Echtzeit, umfassen. Nach weiteren Ausführungsbeispielen kann die Atmungsinformation zusätzlich oder alternativ eine Information über eine lokale Atemkomponente des Patienten umfassen, die angibt, auf welchen Körperteil sich die Atmung des Patienten gerade wie auswirkt. Die Recheneinheit kann einen oder mehrere Prozessoren aufweisen. Die Prozessoren können als zentrale Verarbeitungseinheit (ein englischer Fachausdruck hierfür ist "central processing unit", kurz CPU) ausgebildet sein. Ferner kann die Recheneinheit eine Speichereinrichtung zur zumindest temporären Speicherung von Daten, Signalen und/oder Computerprogrammprodukten umfassen.

Dem vorgeschlagenen differentiellen Spannungsmesssystem liegt die Erkenntnis zugrunde, dass sich aus einer differentiellen Spanungsmessung, wie sie zur Bestimmung der Herzaktivität bereits verwendet wird, bioelektrische Signale ableiten lassen, die durch die Atmungsaktivität des Patienten moduliert sind. Durch die gezielte Gewinnung dieser Signale kann zur Bestimmung der Atmungsaktivität im Prinzip derselbe Setup verwendet werden, wie zur Bestimmung der Herzaktivität des Patienten. Somit wird eine in der Handhabung einfache und was die zusätzlichen Rüstkosten betrifft kostengünstige Lösung bereitgestellt, mit der eine Atmungsaktivität eines Patienten z.B. für eine darauf abgestimmte Bildgebung gewonnen werden kann. Insbesondere eröffnet das vorgeschlagene Spannungsmesssystem die Möglichkeit, Informationen zur Herz- und Atmungsaktivität konzertiert zu erfassen und einer angeschlossenen Bildgebung als integrierte Eingangsinformation, etwa in Form eines Triggersignals bereitzustellen.

Gemäß einigen Ausführungsbeispielen ist die Signalverarbeitungsvorrichtung zur Bestimmung wenigstens zweier verschiedener bioelektrischer Signale aus den Messignalen ausgebildet. Entsprechend ist die Recheneinheit dazu ausgebildet, die wenigstens zwei verschiedenen bioelektrischen Signale jeweils bei der Ermittlung der Atmungsinformation zu berücksichtigen.

Mit anderen Worten werden so zwei unterschiedliche Signale zur Bestimmung der Atmungsinformation herangezogen, was eine verbesserte und sicherere Atmungserkennung gewährleisten kann. Die verschiedenen bioelektrischen Signale können durch jeweils unterschiedliche Signalverarbeitungsstrecken in der Signalverarbeitungsvorrichtung realisiert sein. Beispielsweise können für verschiedene bioelektrische Signale jeweils unterschiedliche Messsignale ausgewählt werden. Zusätzlich oder alternativ können für verschiedene bioelektrische Signale unterschiedliche Signalkomponenten mit entsprechenden Filtermodulen aus den Messsignalen extrahiert werden.

Gemäß einigen Ausführungsbeispielen umfasst das wenigstens eine bioelektrische Signal eine Beat-to-Beat Herzrate des Patienten, einen EKG Vektor des Patienten, und/oder eine Muskelaktivität des Patienten.

Der Verwendung der Beat-to-Beat Herzrate als bioelektrisches Signal liegt die Erkenntnis zugrunde, dass bei Einatmung das Herz schneller schlägt als bei Ausatmung. Zur Bestimmung eines solchen bioelektrischen Signals, kann die Signalverarbeitungsvorrichtung insbesondere ein Filtermodul zur Filterung der Messsignale umfassen, welches Filtermodul zur Filterung von Frequenzen zwischen 1 Hz und 40 Hz ausgebildet ist.

Der Verwendung eines EKG-Vektors als bioelektrisches Signal zur Bestimmung der Atmungsinformation liegt die Erkenntnis zugrunde, dass sich während der Atmung das Herz im Körper verdreht. In dem differentiellen Spannungsmesssystem wird dies dadurch ersichtlich, dass sich die die Amplitude der Messsignale zwischen den Sensorelementen verschiebt bzw. sich die Messsignale ortsabhängig verändern. Zur Messung des EKG-Vektors werden also Messsignale von wenigstens zwei, bevorzugt drei oder mehr, Sensorelementen kombiniert, die bezüglich des Patienten an unterschiedlichen Orten angeordnet sind. Im Wesentlichen werden den Messsignalen zur Bestimmung des EKG-Vektors ähnliche Informationen entnommen, wie bei der Beat-to-Beat Herzrate, nur eben ortsaufgelöst. Entsprechend kann die Signalverarbeitungsvorrichtung zur Bestimmung eines entsprechenden bioelektrischen Signals insbesondere ein Filtermodul zur Filterung der Messsignale umfassen, welches Filtermodul zur Filterung von Frequenzen zwischen 1 Hz und 40 Hz ausgebildet ist.

Der Verwendung eines EKG-Vektors als bioelektrisches Signal zur Bestimmung der Atmungsinformation liegt die Erkenntnis zugrunde, dass durch eine Messung der Muskelaktivität insbesondere im Bereich des Brustkorbs des Patienten direkt auf die Atmungsaktivität des Patienten geschlossen werden kann. Entsprechend kann das differentielle Spannungsmesssystem über wenigstens ein Sensorelement verfügen, dass dazu geeignet ist, und insbesondere so angeordnet ist, dass es den Brustkorb des Patienten in einem Bereich kontaktiert, in dem die Muskelaktivität bei Atmung messbar wird. Die die Muskelaktivität indizierende Messsignale sind durch erhöhte Signalanteile bei 10-100 Hz gekennzeichnet. Entsprechend kann die Signalverarbeitungsvorrichtung zur Bestimmung eines entsprechenden bioelektrischen Signals insbesondere ein Filtermodul zur Filterung der Messsignale umfassen, welches Filtermodul zur Filterung von Frequenzen zwischen 10 Hz und 100 Hz ausgebildet ist.

Gemäß einigen Ausführungsbeispielen kann das wenigstens eine bioelektrische Signal derart ausgebildet sein, dass es eine kapazitive Kopplung zwischen Sensorelement und Patient indiziert. Um ein solches bioelektrisches Signal bereitzustellen kann die Signalverarbeitungsvorrichtung eine Referenzsignalerfassungsvorrichtung und ein Komparatormodul aufweisen. Die Referenzsignalerfassungsvorrichtung ist dazu eingerichtet, ein Referenzsignal zu erfassen. Das Komparatormodul ist dazu eingerichtet, basierend auf dem Referenzsignal ein korrigiertes Messsignal zu ermitteln und basierend auf dem korrigierten Messignal als bioelektrisches Signal ein Signal bereitzustellen, aus dem eine kapazitive Kopplung zwischen zumindest einem Sensorelement und dem Patienten ableitbar ist.

Der Verwendung eines eine kapazitive Kopplung zwischen Sensorelement und Patient indizierenden bioelektrischen Signals als Grundlage zur Ermittlung der Atmungsinformation liegt die Erkenntnis zugrunde, dass sich durch die Atmung des Patienten eine Belastung des Körpers des Patienten auf eine Unterlage ortsabhängig verschiebt. Infolgedessen ändert sich bei den verwendeten kapazitiven Sensorelementen eine Kopplung zwischen Patient und Sensorelektrode, was im Prinzip durch eine Modulation des jeweiligen Messsignals messbar wird. Allerdings wird diese Modulation durch andere Effekte, wie etwa einer Veränderung äußerer elektromagnetischer Felder überlagert. Um solche Überlagerungseffekte zu eliminieren ist vorgesehen, die Messsignale mit einem, insbesondere von außen auf die Sensorelemente einwirkenden, Referenzsignal abzugleichen bzw. gegen ein solches Referenzsignal zu filtern, um so ein entsprechend korrigiertes Messsignal zu erhalten. Die Veränderung der elektrischen Felder auf einzelne Sensorelemente kann so immer in ein Verhältnis zu einem Referenzwert gesetzt werden und spiegelt damit rein die Veränderung der Messsignale dieses Sensorelements durch den veränderten Anpressdruck des Patienten an das Sensorelement wider. Die Referenzsignale werden durch Effekte verursacht, die dem eigentlich zu erfassenden Messgegenstand überlagert sind.

Die Referenzsignalerfassungsvorrichtung kann zur Messung des Referenzsignals beispielsweise über zusätzliche Sensoren verfügen, und/oder dazu ausgebildet sein, das Referenzsignal durch Messung von elektrischen Strömen oder Spannungen an oder in dem differentiellen Spannungsmesssystem selbst zu erfassen. Die Referenzsignalerfassungsvorrichtung kann beispielsweise das bereits erwähnte dritte kapazitive Sensorelement umfassen.

Gemäß einigen Ausführungsbeispielen umfasst das Komparatormodul einen Referenz-Formungsfilter welcher dazu eingerichtet ist, das Referenzsignal zu filtern, und eine adaptiven Filtereinheit, welche dazu eingerichtet ist, das Messsignal auf Basis des gefilterten Referenzsignals zu filtern und so das korrigierte Messsignal zu ermitteln.

Der Referenz-Formungsfilter, kann beispielsweise als Tiefpassfilter ausgebildet sein. Vorteilhaft werden hochfrequente Anteile des Referenzsignals, die, anders als bei einer Messung mit Elektroden, bei einer Messung mit kapazitiven Messsensoren, was Frequenz und Phase betrifft, nicht gut zu den Störungen des Messsignals passen, unterdrückt. Nach diesem Filtervorgang umfasst das Referenzsignal vor allem niederfrequente Anteile, die gut mit den Referenzsignal-Anteilen auf dem Messsignal übereinstimmen.

Der Eingang des adaptiven Filterelements ist dem Referenz-Formungsfilter nachgeschaltet, damit das Komparatormodul das bereits durch den Referenz-Formungsfilter gefilterte Referenzsignal als Referenzsignal weiterverarbeiten kann. Bei diesem adaptiven Filtervorgang erfolgt eine Schätzung eines Referenzsignal-Anteils des Messsignals auf Basis des von dem Referenz-Formungsfilter gefilterten Referenzsignals. Dieser Referenzsignal-Anteil wird dann aus dem Messsignal entfernt. Die Adaption des adaptiven Filters kann über iterative oder rekursive Optimierungsprozesse erfolgen. Auf diese Weise werden insbesondere niederfrequente Anteile des Referenzsignals, also das gefilterte Referenzsignal, für einen adaptiven Filterprozess genutzt, um Referenzsignal-Anteile zu entfernen und eine Modulation des Signals durch einen veränderten Kontakt des Patienten mit dem Sensorelement zu isolieren.

Gemäß einigen Ausführungsbeispielen weist die Signalverarbeitungsvorrichtung einen Vorfilter, insbesondere in Form eines Kammfilters, auf, welcher Vorfilter dazu ausgebildet ist, aus dem Messignal und/oder dem Referenzsignal Frequenzen von 50Hz, 60Hz und/oder deren Harmonische zu extrahieren.

Die Verwendung eines Referenzsignals und die Analyse des Messsignals in diesen Frequenzen hat den Vorteil, dass in einer elektrisch betriebenen Umgebung immer elektrische Felder der Netz-Spannungsversorgung bei 50Hz bzw. 60 Hz und deren harmonischen Frequenzen für diese Analyse vorliegen. Es ist folglich nicht nötig, ein eigenes Referenzsignal aufzuprägen.

Gemäß einigen Ausführungsbeispielen ist das wenigstens eine bioelektrische Signal ein ortsaufgelöstes Signal. Dies kann insbesondere bedeuten, dass Signalanteile auf einzelne Sensorelemente zurückgeführt werden können und so eine Aussage über Verhältnisse am Ort des jeweiligen Sensors ermöglichen. Ortsaufgelöste bioelektrische Signale können insbesondere der EKG-Vektor, das Muskelaktivitätssignal und/oder das eine kapazitive Kopplung indizierende Signal sein.

Gemäße einigen Ausführungsbeispielen weist das differentielle Spannungsmesssystem ferner eine Anzahl drucksensitiver Messelemente auf, die jeweils zur Erfassung eines eine lokale Druckbelastung durch den Patienten auf eine Unterlage indizierenden Drucksignals ausgebildet sind. Ferner weist das differentielle Spannungsmesssystem eine sekundäre Signalverarbeitungsvorrichtung zur Bestimmung wenigstens eines, insbesondere ortsaufgelösten, Druckbelastungssignals aus den Drucksignalen auf, wobei die Recheneinheit ferner dazu ausgebildet ist, die eine Atmungsaktivität indizierende Information zusätzlich basierend auf dem Druckbelastungssignal bereitzustellen.

Durch die Bereitstellung von drucksensitiven Messelementen und der Auswertung der durch diese erfassten Messsignale kann eine zur differentiellen Spannungsmessung orthogonale Information erhalten werden, mit der sich die Atmungsinformation genauer bestimmen lässt. Da das Gewicht des Patienten sich bei der Atmung verschiebt, werden unterschiedliche Messelemente unterschiedlich stark komprimiert. Insbesondere lässt sich dadurch eine lokale Atmungskomponente des Patienten bestimmen, die nicht nur die Atemfrequenz angibt, sondern auch anzeigt welches Körperteil von der Atmung betroffen ist.

Die Unterlage kann dabei beispielsweise als Patientenmatte ausgebildet sein. Die drucksensitiven Messelemente und/oder die kapazitiven Messelemente können dabei an und/oder in der Unterlage angeordnet bzw. in diese eingearbeitet sein. Gemäß einigen Ausführungsbeispielen ist die Unterlage in dem differentiellen Spannungsmesssystem umfasst.

Gemäß einigen Ausführungsbeispielen weist wenigstens eines der kapazitiven Sensorelemente eine mechanische Aufhängung auf, welche mechanische Aufhängung dazu ausgebildet ist, eine Sensorelektrode des entsprechenden Sensorelements, insbesondere in der Unterlage, abzustützen, wobei in der mechanischen Aufhängung zusätzlich eines der drucksensitiven Messelemente aufgenommen ist.

Durch diese Ausführung können drucksensitive Messelemente einfach integriert werden und liefern zudem ein ergänzendes Drucsignal vom Ort des kapazitiven Sensorelements, was z.B. eine Verifikation der Messsignale durch einen Abgleich mit einem entsprechenden Drucsignal gestatten kann. Zusätzlich oder alternativ können freilich weitere drucksensitive Messelemente unabhängig von den kapazitiven Sensorelementen bereitgestellt werden.

Gemäß einigen Ausführungsbeispielen weist die mechanische Aufhängung ein Trägerelement auf, das, insbesondere elastisch, deformierbar und bevorzugt aus Schaumstoff ausgebildet ist und die Sensorelektrode abstützt. Das drucksensitive Messelement umfasst zwei leitfähige Schichten, wobei die eine Schicht auf einer ersten Seite des Trägerelements angeordnet ist, und die andere Schicht auf einer der ersten Seite gegenüberliegenden zweiten Seite des Trägerelements angeordnet ist. Ferner umfasst das differentielle Spannungsmesssystem eine Druckmessschaltung, die dazu ausgebildet ist, die leitfähigen Schichten mit einem konstanten Strom zu beaufschlagen und die dadurch abfallende Spannung als Drucsignal zu erfassen. Auch möglich ist freilich ein separates drucksensitives Messelement unter oder auf dem Trägerelement.

Gemäß einigen Ausführungsbeispielen weist das differentielle Spannungsmesssystem ferner eine Ausgabeeinheit auf, die dazu eingerichtet ist, die eine Atmungsaktivität indizierenden Information und wenigstens eines der bioelektrischen Signale auszugeben, wobei das ausgegebene bioelektrische Signal einen Herzschlag des Patienten indiziert. Entsprechend werden durch das differentielle Spannungsmesssystem konzertierte Informationen von sowohl der Herz- als auch der Atmungsaktivität des Patienten bereitgestellt, die sich beispielsweise verwenden lassen, um nachgeschaltete Systeme und/oder Anwendungen mit der Herz- und/oder Atmungsaktivität zu synchronisieren.

Gemäß einigen Ausführungsbeispielen umfasst die Recheneinheit ein Kalman-Filter, das dazu ausgebildet ist, basierend auf den in die Recheneinheit eingegebenen Signalen (also insbesondere das oder die bioelektrischen Signale und/oder das Druckbelastungssignal) die eine Atmungsaktivität des Patienten indizierende Information zu ermitteln und bereitzustellen. Die Verwendung eines Kalman-Filters ermöglicht dabei insbesondere, die Atmungsinformation aus den Eingangssignalen in Echtzeit abzuschätzen.

Gemäß einigen Ausführungsbeispielen ist die Recheneinheit dazu ausgebildet, eine trainierte Funktion auf die in die Recheneinheit eingegebenen Signale anzuwenden, welche trainierte Funktion dazu ausgebildet ist, basierend auf den in die Recheneinheit eingegebenen Signalen die eine Atmungsaktivität des Patienten indizierende Information zu ermitteln und bereitzustellen.

Eine trainierte Funktion bildet allgemein Eingabedaten auf Ausgabedaten ab. Hierbei können die Ausgabedaten insbesondere von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen Parameters oder der mehreren Parameter der trainierten Funktion kann insbesondere auf einem Paar aus Trainingseingabedaten und zugehörigen Trainingsausgabedaten basieren, wobei die trainierte Funktion zur Erzeugung von intermediären Ausgabedaten auf die Trainingseingabedaten angewendet wird. Insbesondere können das Bestimmen und/oder das Anpassen auf einem Vergleich der intermediären Ausgabedaten und der Trainingsausgabedaten basieren. Im Allgemeinen wird auch eine trainierbare Funktion, d.h. eine Funktion mit noch nicht angepassten Parametern, als trainierte Funktion bezeichnet. Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens.

Gemäß einigen Ausführungsbeispielen umfasst die trainierte Funktion ein künstliches neuronales Netzwerk. Anstatt des Begriffs "neuronales Netzwerk" kann auch der Begriff "neuronales Netz" verwendet werden. Ein neuronales Netzwerk ist im Grunde genommen wie ein biologisches neuronales Netz - etwa ein menschliches Gehirn - aufgebaut. Insbesondere umfasst ein künstliches neuronales Netzwerk eine Eingabeschicht und eine Ausgabeschicht. Es kann ferner mehrere Schichten zwischen Eingabe- und Ausgabeschicht umfassen. Jede Schicht umfasst mindestens einen, vorzugsweise mehrere, Knoten. Jeder Knoten kann als biologische Verarbeitungseinheit verstanden werden, z.B. als Neuron. Mit anderen Worten entspricht jedes Neuron einer Operation, die auf Eingabedaten angewendet wird. Knoten einer Schicht können durch Kanten oder Verbindungen mit Knoten anderer Schichten verbunden sein, insbesondere durch gerichtete Kanten oder Verbindungen. Diese Kanten oder Verbindungen definieren den Datenfluss zwischen den Knoten des Netzwerks. Die Kanten oder Verbindungen sind mit einem Parameter assoziiert, der häufig als "Gewicht" oder "Kantengewicht" bezeichnet wird. Dieser Parameter kann die Wichtigkeit der Ausgabe eines ersten Knotens für die Eingabe eines zweiten Knotens regulieren, wobei der erste Knoten und der zweite Knoten durch eine Kante verbunden sind. Insbesondere kann eine trainierte Funktion auch ein tiefes künstliches neuronales Netzwerk aufweisen (englischer Fachbegriff sind "deep neural network" oder "deep artificial neural network"). Insbesondere kann ein neuronales Netzwerk trainiert werden. Insbesondere wird das Training eines neuronalen Netzwerks basierend auf den Trainingseingabedaten und den zugehörigen Trainingsausgabedaten gemäß einer "überwachten" Lerntechnik (ein englischer Fachbegriff ist "supervised learning") durchgeführt, wobei die bekannten Trainingseingabedaten in das neuronale Netzwerk eingegeben und die vom Netzwerk generierten intermediären Ausgabedaten mit den zugehörigen Trainingsausgabedaten verglichen werden. Das künstliche neuronale Netzwerk lernt und passt die Kantengewichte für die einzelnen Knoten unabhängig an, solange die intermediären Ausgabedaten der letzten Netzwerkschicht den Trainingsausgabedaten nicht ausreichend entsprechen.

Gemäß einem Aspekt wird ein Computer-implementiertes Verfahren zur Bereitstellung einer trainierten Funktion bereitgestellt. Das Verfahren weist mehrere Schritte auf. Ein erster Schritt ist auf ein Bereitstellen eines oder mehrerer bioelektrischer Trainings-Signale und, optional, eines Trainings-Druckbelastungssignals eines Patienten als Trainingseingabedaten gerichtet. Ein weiterer Schritt ist auf ein Bereitstellen einer Trainings-Atmungsinformation gerichtet, die eine zu den bioelektrischen Trainings-Signalen und/oder Trainings-Druckbelastungssignalen entsprechende Atmungsaktivität des Patienten indiziert. Ein weiterer Schritt ist auf ein Anwenden der trainierten Funktion auf die Trainingseingabedaten gerichtet, um eine intermediäre Atmungsinformation zu erzeugen. Ein weiterer Schritt ist auf ein Vergleichen der intermediären Atmungsinformation mit der Trainings-Atmungsinformation gerichtet. Ein weiterer Schritt ist auf ein Anpassen der trainierten Funktion basierend auf dem Vergleich gerichtet. Ein weiterer Schritt ist auf ein Bereitstellen der trainierten Funktion gerichtet.

Gemäß einigen Ausführungsbeispielen werden die bioelektrischen Trainings-Signale bzw. die Trainings-Druckbelastungssignals mit einem differentiellen Spannungsmesssystem gemäß erfindungsgemäßen Ausführungsbeispielen erzeugt und die Trainingsatmungsinformation wird parallel dazu mit einem Messsystem erzeugt, das auf einem von einer differentiellen Spannungsmessung mit kapazitiven Sensorelementen verschiedenen Messverfahren beruht, und insbesondere auf einem Messverfahren unter Verwendung eines Atemgurts und/oder einem Kamera- oder Radar-basierten Messverfahren beruht.

Gemäß einem Aspekt wird ein Verfahren zur Bereitstellung einer eine Atmung eines Patienten indizierenden Information, insbesondere mit einem differentiellen Spannungsmesssystem gemäß erfindungsgemäßen Ausführungsbeispielen, bereitgestellt. Das Verfahren weist mehrere Schritte auf. Ein erster Schritt ist auf ein Erfassen einer Anzahl auf den Patienten bezogener Messignale mit wenigstens zwei kapazitiven Sensorelementen gerichtet. Ein weiterer Schritt ist auf ein Bestimmen wenigstens eines bioelektrischen Signals aus den Messignalen gerichtet. Ein weiterer Schritt ist auf eine Ermittlung der eine Atmung des Patienten indizierenden Information basierend auf dem bestimmten wenigsten einen bioelektrischen Signal gerichtet.

Die bezüglich den Ausführungsbeispielen des differentiellen Spannungsmesssystems offenbarten Vorteile und Weiterbildungen lassen sich auf das obige Verfahren übertragen. Insbesondere können Elemente des differentiellen Spannungsmesssystems und deren Ausgestaltungen in entsprechende Verfahrensschritte umgesetzt werden.

Gemäß einigen Ausführungsbeispielen umfasst das Verfahren ferner die Schritte: ein Erfassen von eine Druckbelastung des Patienten auf eine Unterlage indizierenden Drucksignalen mit wenigstens einem drucksensitiven Messelement sowie ein Bestimmen wenigstens eines, insbesondere ortsaufgelösten, Druckbelastungssignals aus den Drucksignalen, wobei der Schritt des Ermittelns der eine Atmung des Patienten indizierenden Information zusätzlich basierend auf dem Druckbelastungssignal erfolgt.

Die Erfindung betrifft in einem weiteren Aspekt ein Computerprogrammprodukt, mit einem Computerprogramm, welches direkt in eine Speichereinrichtung eines differentiellen Spannungsmesssystems ladbar ist, mit Programmabschnitten, um alle Schritte der Verfahren nach den obigen Ausführungsbeispielen auszuführen, wenn das Computerprogramm in dem differentiellen Spannungsmesssystem ausgeführt wird.

Ferner betrifft die Erfindung in einem weiteren Aspekt ein computerlesbares Speichermedium, auf welchem von einer Recheneinheit einlesbare und ausführbare Programmabschnitte gespeichert sind, um alle Schritte der Verfahren nach den obigen Ausführungsbeispielen auszuführen, wenn die Programmabschnitte von der Recheneinheit ausgeführt werden.

Die Computerprogrammprodukte können dabei eine Software mit einem Quellcode, der noch kompiliert und gebunden oder der nur interpretiert werden muss, oder einen ausführbaren Softwarecode umfassen, der zur Ausführung nur noch in die Recheneinheit zu laden ist. Durch die Computerprogrammprodukte können die Verfahren schnell, identisch wiederholbar und robust ausgeführt werden. Die Computerprogrammprodukte sind so konfiguriert, dass sie mittels der Recheneinheit die erfindungsgemäßen Verfahrensschritte ausführen können. Die Recheneinheit muss dabei jeweils die Voraussetzungen wie beispielsweise einen entsprechenden Arbeitsspeicher, einen entsprechenden Prozessor, eine entsprechende Grafikkarte oder eine entsprechende Logikeinheit aufweisen, sodass die jeweiligen Verfahrensschritte effizient ausgeführt werden können.

Die Computerprogrammprodukte sind beispielsweise auf einem computerlesbaren Speichermedium gespeichert oder auf einem Netzwerk oder Server hinterlegt, von wo sie in den Prozessor der jeweiligen Recheneinheit geladen werden können, der mit der Recheneinheit direkt verbunden oder als Teil der Recheneinheit ausgebildet sein kann. Weiterhin können Steuerinformationen der Computerprogrammprodukte auf einem computerlesbaren Speichermedium gespeichert sein. Die Steuerinformationen des computerlesbaren Speichermediums können derart ausgebildet sein, dass sie bei Verwendung des Datenträgers in einer Recheneinheit ein erfindungsgemäßes Verfahren durchführen. Beispiele für computerlesbaren Speichermedium sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemäßen Ausführungsformen/Aspekte der vorab beschriebenen Verfahren durchgeführt werden. So kann die Erfindung auch von dem besagten computerlesbaren Medium und/oder dem besagten computerlesbaren Speichermedium ausgehen. Die Vorteile der vorgeschlagenen Computerprogrammprodukte bzw. der zugehörigen computerlesbaren Medien entsprechen im Wesentlichen den Vorteilen der vorgeschlagenen Verfahren.

Weitere Besonderheiten und Vorteile der Erfindung werden aus den nachfolgenden Erläuterungen von Ausführungsbeispielen anhand von schematischen Zeichnungen ersichtlich. In diesem Zusammenhang genannte Modifikationen können jeweils miteinander kombiniert werden, um neue Ausführungsformen auszubilden. In unterschiedlichen Figuren werden für gleiche Merkmale die gleichen Bezugszeichen verwendet.

Es zeigen:

Fig. 1 eine Ansicht eines differentiellen Spannungsmesssystems gemäß einem Ausführungsbeispiel;

Fig. 2 eine Ansicht eines differentiellen Spannungsmesssystems mit Signalmessschaltungen gemäß einem weiteren Ausführungsbeispiel;

Fig. 3 ein beispielhaftes Signalverarbeitungsmodul einer Signalverarbeitungsvorrichtung gemäß einem Ausführungsbeispiel;

Fig. 4 eine Ansicht eines differentiellen Spannungsmesssystems gemäß einem weiteren Ausführungsbeispiel;

Fig. 5 eine Ansicht eines Sensorelements gemäß einem Ausführungsbeispiel;

Fig. 6 eine Ansicht eines Sensorelements gemäß einem weiteren Ausführungsbeispiel;

Fig. 7 ein Ablaufdiagramm eines Verfahrens zur Bestimmung einer Atmungsaktivität eines Patienten gemäß einem Ausführungsbeispiel;

Fig. 8 mit einem Verfahren oder System zur Bestimmung einer Atmungsaktivität eines Patienten assoziierte Signalpfade gemäß einem Ausführungsbeispiel;

Fig. 9 eine Ansicht einer trainierten Funktion gemäß einem Ausführungsbeispiel; und

Fig. 10 ein Ablaufdiagramm eines Verfahrens zur Bereitstellung einer trainierten Funktion gemäß einem Ausführungsbeispiel.

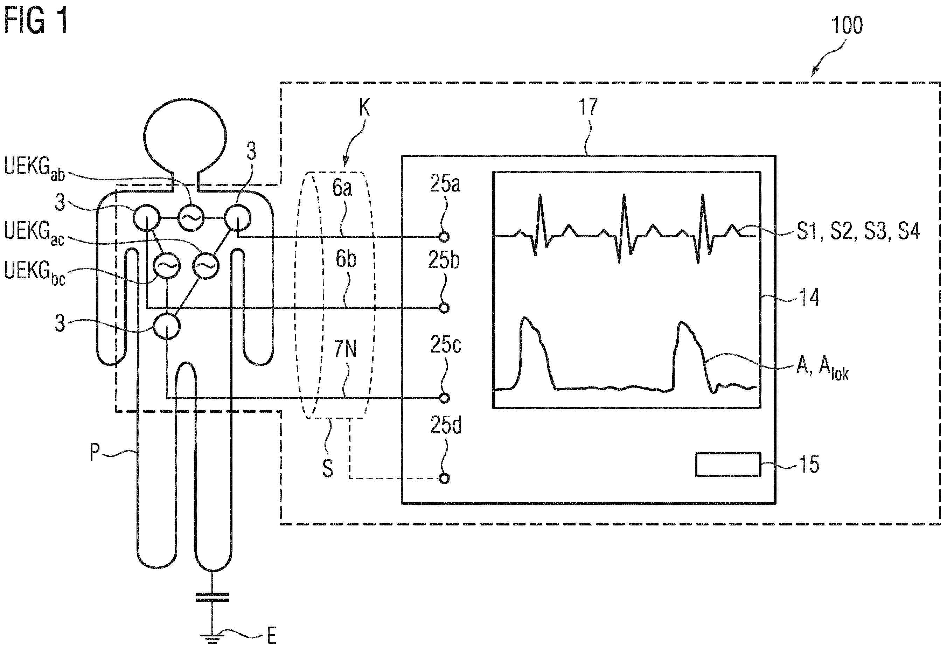

Figur 1 zeigt eine Ansicht eines an einem Patienten P angeordneten, differentiellen Spannungsmesssystems 100. Das Spannungsmesssystem 100 umfasst ein kombiniertes EKG- und Atmungserkennungs-Gerät 17 mit seinen elektrischen Anschlüssen und daran über Kabel K angeschlossene Sensorelemente 3 um an dem Patienten P bioelektrische Signale S1, S2, S3, S4 zu ermitteln. Wenigstens eine, bevorzugt alle Sensorelemente 3, können als Teil eines erfindungsgemäßen Spannungsmesssystems 100 wie in weiteren Figuren beschrieben, ausgebildet sein.

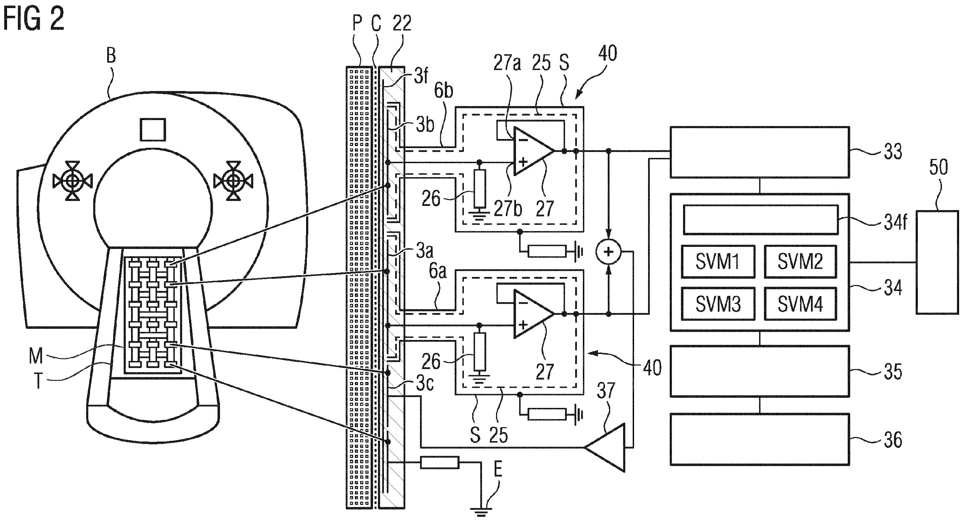

Um die bioelektrische Signale S1, S2, S3, S4 zu ermitteln, werden mindestens ein erstes Sensorelement 3 und ein zweites Sensorelement 3 benötigt, die jeweils eine Sensorelektrode 3a, 3b aufweisen (vgl. Figur 2) die an, auf oder unter dem Patienten P angebracht sind. Durch Signalmesskabel K sind die Sensorelektroden 3a, 3b über Anschlüsse 25a, 25b, meist Steckverbindungen, mit dem Gerät 17 verbunden. Die erste Elektrode 3a und die zweite Elektrode 3b einschließlich der Signalmesskabel K bilden dabei einen Teil einer Signalerfassungseinheit, mit der die bioelektrische Signale S1, S2, S3, S4 erfasst werden können.

Eine drittes Sensorelement 3 weist eine dritte Sensorelektrode 3c auf, die als Referenzelektrode dient, um einen Potentialausgleich zwischen dem Patienten P und dem Gerät 17 zu schaffen. Klassisch wird diese dritte Elektrode 3c am rechten Bein des Patienten angebracht ("Right-Leg-Drive" bzw. "RLD"). Sie kann aber auch an einer anderen Stelle positioniert werden. Darüber hinaus können über weitere Anschlüsse, welche nicht dargestellt sind, am Gerät 17 noch eine Vielzahl weiterer Kontakte für weitere Ableitungen (Potentialmessungen) am Patienten P angebracht und können für die Bildung von geeigneten Signalen genutzt werden.

Zwischen den einzelnen Elektroden 3a, 3b, 3c bilden sich die Spannungspotentiale UEKGab, UEKGbc und UEKGac, die zur Messung der bioelektrische Signale S1, S2, S3, S4 dienen.

Die ermittelten bioelektrische Signale S1, S2, S3, S4 können auf einer Benutzerschnittstelle 14 des Geräts 17 angezeigt werden. Diese können z.B. ein EKG-Signal, ein EKG-Vektor oder eine Muskelaktivität umfassen. Zusätzlich können auf der Benutzerschnittstelle 14 noch Atmungsaktivitäts-Signale bzw. Atmungsinformationen A, Alok angezeigt werden, die aus den bioelektrischen Signalen S1, S2, S3, S4 und optional weiteren Messgrößen abgeleitet werden. Eine Atmungsinformation A, Alok kann beispielsweise eine Information A über Ein-/Ausatemzyklen des Pateinten P oder eine Information Alok über eine lokale Atmungskomponente umfassen, welche lokale Atmungskomponente angibt, wie sich die Atmung auf verschiedene Körperpartien ausgewirkt hat.

Der Patient P ist bei der Messung zumindest kapazitiv mit dem Erdpotential E gekoppelt (durch eine Kopplung am rechten Bein dargestellt).

Die Signalmesskabel K, welche von der ersten Sensorelektrode 3a und der zweiten Sensorelektrode 3b zum Gerät 17 führen, sind ein Teil der Nutzsignalpfade 6a, 6b bzw. der entsprechenden Sensorleitungen. Das Signalmesskabel K, welches von der Elektrode 3c zum Gerät 17 führt, entspricht hierbei einem Teil eines dritten Nutzsignalpfads 7N. Der dritte Nutzsignalpfad 7N kann insbesondere dazu dienen, Störsignale, welche über den Patienten P und die Elektroden eingekoppelt wurden, zu übertragen.

Die Kabel K weisen eine Schirmung S auf, die hier schematisch als ein alle Nutzsignalpfade 6a, 6b, 7N umgebender gestrichelter Zylinder dargestellt ist. Die Schirmung muss aber nicht alle Kabel K gemeinsam umgeben, sondern die Kabel K können auch separat geschirmt sein. Die Anschlüsse 25a, 25b, 25c weisen jedoch bevorzugt jeweils integriert einen Pol für die Schirmung S auf. Diese Pole werden dann auf einen gemeinsamen Schirmungsanschluss 25d zusammengeführt. Die Schirmung S ist dabei z.B. als eine den Leiter des jeweiligen Kabels K umgebende Metallfolie ausgebildet, die jedoch von dem Leiter isoliert ist.

Zudem kann das Gerät 17 eine externe Schnittstelle 15 aufweisen, um beispielsweise einen Anschluss für einen Drucker, eine Speichereinrichtung und/oder sogar ein Netzwerk bereitzustellen. Das Gerät 17 weist den jeweiligen Anschlüssen 25a, 25b zugeordnete Signalmessschaltungen 40 (siehe z.B. Figur 2) gemäß Ausführungsbeispielen der Erfindung auf. Die Signalmessschaltungen 40 sind jeweils wieder über einen Erdschalter 31 mit Erde E verbunden.

Figur 2 zeigt eine Ansicht eines differentiellen Spannungsmesssystems 100 beispielhaft umfassend zwei Signalmessschaltungen 40 gemäß einem Ausführungsbeispiel. Die beiden Signalmessschaltungen 40 weisen einen identischen Aufbau auf, weswegen sich entsprechende Komponenten der Übersichtlichkeit halber weitgehend nur einmal mit Bezugszeichen versehen wurden.

Die Anordnung eines einzelnen Sensors 3 bzw. einer einzelnen Sensorelektrode 3a der Signalmessschaltung 40 ist hier in Form einer kapazitiven Messschaltung veranschaulicht. Patient P und Sensorelektrode 3a befinden sich in räumlicher Nähe zueinander. Genauer gesagt liegt Patient P hier auf einem Patiententisch T einer Bildgebungsanlage B in Form einer Computertomographie-Anlage. Auf dem Patiententisch T ist eine kapazitive Patientenmatte M angeordnet, auf welcher der Patient P positioniert ist. Die Patientenmatte M umfasst eine Vielzahl von erfindungsgemäßen Signalmessschaltungen 40. Die Matte M kann alternativ als Elektroden-Pad ausgebildet sein, welches insbesondere in einer Rückenlehne eines Untersuchungs- oder Behandlungsstuhls angeordnet sein kann. Zwei der Signalmessschaltungen 40 werden im Folgenden näher erläutert. Patient P kann bspw. mit einer Stoffbekleidung C versehen sein. Darüber liegt optional eine für Röntgenstrahlen transparente Decke 22. Die Sensorelektroden 3a, 3b, 3c stehen mit dem Patienten P nicht in direktem, elektrischen Kontakt, sondern er wird mindestens von einer Sensorabdeckung 3f von dem Patienten P elektrisch isoliert. Allerdings wird eine kapazitive Einkopplung eines Signals durch die Sensorabdeckung 3f nicht beeinträchtigt. Die Sensorelektrode 3a, 3b, 3c eine von der Sensorelektrode 3a, 3b, 3c zu einem Operationsverstärker 27 verlaufende Sensorleitung 6a sowie die den Operationsverstärker 27 umfassende Messschaltung 40 werden von einem sogenannten aktiven Schutzschirm 25 sowie bevorzugt einer Schirmung S umgeben.

Der Operationsverstärker 27 ist als sogenannter Nachläufer ausgebildet. D.h., der negative Eingang 27a des Operationsverstärkers 27 ist mit dem Ausgang 28 des Operationsverstärkers 27 gekoppelt. Auf diese Weise wird für den Operationsverstärker 27 am positiven Eingang 27b eine hohe virtuelle Eingangsimpedanz erzielt. Damit ist gemeint, dass aufgrund der Spannungsanpassung zwischen dem Ausgang 28 und dem positiven Eingang 27b kaum ein Strom zwischen dem Sensorelement 3 und dem aktiven Schutzschirm 25 fließt. Weiterhin wird der positive Eingang 27b des Operationsverstärkers 27 mit Hilfe eines gegen die Messgerätmasse (auch als "Messground" bezeichnet) geschalteten Widerstands 26 auf einer elektrischen Biasspannung gehalten. Damit lässt sich der positive Eingang auf ein gewünschtes Messpotential setzen. Auf diese Weise werden DC-Anteile unterdrückt. Dies ist erwünscht, da die Sensorelektrode 3a, 3b, 3c vor allem kapazitiv koppeln und ein sich änderndes Potential vermieden werden soll.

Das in den entsprechenden Verstärkerschaltungen gespeicherte Messsignal MS wird durch einen AD-Wandler (nicht gezeigt) digitalisiert und durch eine Schaltmatrix 33 an eine Signalverarbeitungsvorrichtung 34 übermittelt.

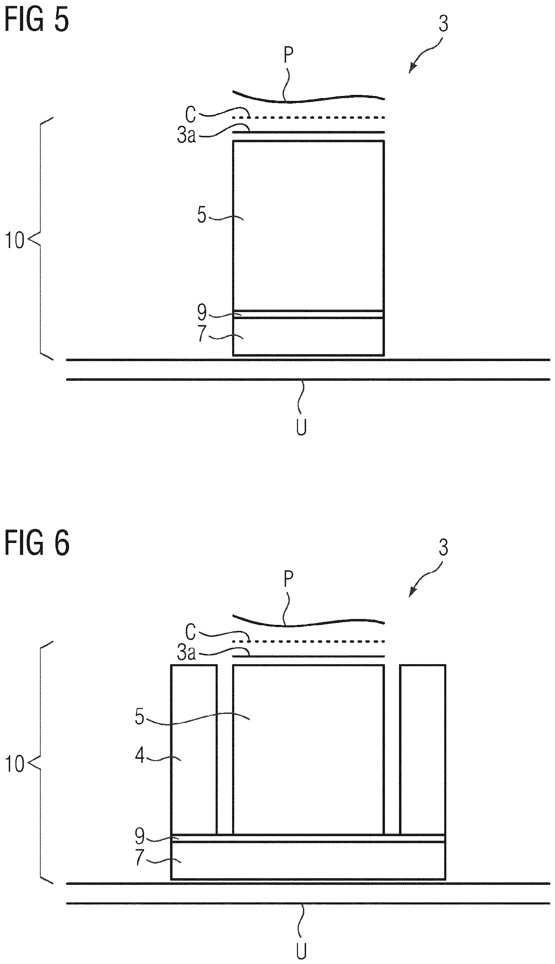

Die gezeigten Signalmessschaltungen 40 umfassen jeweils ein Sensorelement 3, welches beispielsweise wie in Figur 5 dargestellt mit einer mechanischen Aufhängung 10 ausgebildet sein kann. Active Guard 25 und Shield S umschließen jeweils die Sensorelektrode 3, um diese wirksam abzuschirmen. Active Guard 25 und Shield S umschließen ferner die Sensorleitung 6a und durchsetzen gemeinsam mit ihr die mechanische Aufhängung 10 auf dem Weg zum Operationsverstärker 27. Alternative Anordnungen der Sensorleitung 6a sind natürlich ebenfalls denkbar. Die Signalmessschaltungen 40 liefern also kapazitive Messsignale MS, die insbesondere durch eine EKG-Aktivität des Patienten P moduliert sind. Gemäß einigen Ausführungsformen können die Messsignale MS daher auch EKG-Messsignale genannt werden.

Eine weitere Elektrode ist auch in der hier gezeigten Patientenmatte M zur zumindest kapazitiven Kopplung des Patienten an das Erdpotential E vorgesehen.

Eine weitere Elektrode bzw. die dazugehörige Messschaltung 37 fungiert in der Patientenmatte M als Referenzelektrode, bspw. als sog. Driven Neutral Electrode (DNE).

Das differentielle Spannungsmesssystem 100 umfasst ferner eine Schaltvorrichtung in Form einer Switch Matrix 33. Bei einer Vielzahl von Sensorelektroden 3 dient sie dazu, auszuwählen, welche Messsignale MS der Sensorelektroden für eine weitere Signalverarbeitung verwendet werden.

Das differentielle Spannungsmesssystem 100 umfasst ferner eine Signalverarbeitungsvorrichtung 34 z.B. in Form einer sog. Signal Processing Box. Diese ist ausgebildet, eine Vorverarbeitung der erfassten Messsignale MS durchzuführen, um Störanteile zu entfernen und/oder die Messsignale MS geeignet zu filtern und/oder zu kombineren. Die Signalverarbeitungsvorrichtung 34 kann ausgebildet sein, eine Verarbeitung mit frequenzbasiertem Vorfilter 34f wie Bandpass-, Bandstop-, Hochpass-, Tiefpass- oder Kammfilter aber auch eine erweiterte Störunterdrückung wie bspw. in der deutschen Patentanmeldung DE 102019203627A auszuführen. Der Vorfilter 34f kann unterschiedliche Filtermodule aufweisen, um je nach aus den Messsignalen MS abzuleitenden bioelektrischen Signalen S1, S2, S3, S4 geeignete Signalanteile herauszufiltern und unerwünschte Signalanteile zu unterdrücken. Die Filtermodule können durch physische Module und/oder virtuelle, insbesondere software-basierte, Module realisiert sein.

Die Signalverarbeitungsvorrichtung 34 ist insbesondere dazu ausgebildet, aus den Eingangsgrößen umfassend die Messsignale MS eines oder mehrere bioelektrische Signale S1, S2, S3, S4 abzuleiten. Zur Berechnung der bioelektrischen Signale S1, S2, S3, S4 kann die Signalverarbeitungsvorrichtung 34 jeweils Signalverarbeitungsmodule SVM1, SVM2, SVM3, SVM4 aufweisen. Die vorgenommene Unterteilung in Signalverarbeitungsmodule SVM1, SVM2, SVM3, SVM4 dient dabei lediglich der einfacheren Erklärung der Funktionsweise der Signalverarbeitungsvorrichtung 34 und ist nicht beschränkend zu verstehen. Die Signalverarbeitungsmodule SVM1, SVM2, SVM3, SVM4 bzw. deren Funktionen können auch in einem Element zusammengefasst sein. Die Signalverarbeitungsmodule SVM1, SVM2, SVM3, SVM4 können dabei insbesondere auch als Computerprogrammprodukte oder Computerprogrammsegmente aufgefasst werden, welche bei der Ausführung in der Signalverarbeitungsvorrichtung 34 ein oder mehrere der nachstehend beschriebenen Funktionen oder Verfahrensschritte realisieren.

Gemäß einer Ausführungsform umfassen die bioelektrische Signale S1, S2 EKG-Signale des Patienten P. Hierfür sind insbesondere Frequenzen zwischen 1 und 40 Hz relevant. Deshalb kann der Vorfilter 34f ein entsprechendes Filtermodul aufweisen, das derartige Frequenzen aus den Messsignalen MS filtert und weitergibt. Ein solches Filtermodul kann beispielsweise als digitaler Bandpass- und Notch-Filter ausgebildet sein.

Basierend auf einer solchen Vorverarbeitung kann z.B. ein Beat-to-Beat Herzschlag des Patienten P als bioelektrisches Signal S1 abgeleitet werden. Hierfür kann die Signalverarbeitungsvorrichtung 34 über ein Herzschlag-Signalverarbeitungsmodul SVM1 verfügen, das dazu ausgebildet ist, in den insbesondere vorverarbeiteten Messsignalen MS einen Herzschlag des Patienten P zu erkennen. Hinsichtlich einer anschließenden Atmungserkennung liegt dem die Überlegung zugrunde, dass bei Einatmung das Herz des Patienten P schneller und bei Ausatmung langsamer schlägt.

Ferner kann aus den, insbesondere durch Filterung im Bereich 1 bis 40 Hz vorverarbeiteten, Messsignalen MS ein EKG-Vektor-Signal S2 ermittelt werden. Hierzu werden Messsignale MS einer Vielzahl von Sensorelementen 3 in einem entsprechenden EKG-Vektor-Signalverarbeitungsmodul SVM2 der Signalverarbeitungsvorrichtung 34 ausgewertet. Dem liegt die Überlegung zugrunde, dass sich während der Atmung das Herz im Körper verdreht, wodurch sich auch die von außen gemessenen, ortsabhängigen EKG-Signale verändern. Mit anderen Worten dreht sich dabei ein aus mehreren Signalen kombinierter EKG-Vektor. Vereinfacht ausgedrückt verschiebt sich die Amplitude der EKG-Signale zwischen den Sensorelementen 3.

Gemäß weiteren Beispielen kann aus den Messsignalen MS ein bioelektrisches Signal S3 gewonnen werden, das durch eine Muskelaktivität des Patienten moduliert wird. Dem liegt die Überlegung zugrunde, dass gerade bei einer Vielzahl von Sensorelementen 3 es sehr wahrscheinlich ist, dass eines oder mehrere Sensorelemente 3 den Brustkorb des Patienten P in einem Bereich kontaktieren, in dem die Muskelaktivität bei Atmung des Patienten P messbar wird. Da hierfür Signalanteile von 10Hz bis 100Hz relevant sind, kann der Vorfilter 34f über ein entsprechendes Filtermodul verfügen, das z.B. als digitaler Bandpass- und Notch-Filter ausgebildet sein kann. Die Signalverarbeitungsvorrichtung 34 kann entsprechend über ein Muskelaktivitäts-Signalverarbeitungsmodul SVM3 verfügen, das dazu ausgebildet ist, basierend auf dem ggf. durch den Vorfilter 34f geeignet vorbearbeiteten Messsignalen MS eine Muskelaktivität des Patienten zu bestimmen.

Gemäß weiterer Beispielen kann aus den Messsignalen MS ein bioelektrisches Signal S4 gewonnen werden, das durch die kapazitive Kopplung des Patienten P mit den Sensorelementen 3 moduliert wird bzw. eine solche Kopplung indiziert. Die Abbildung elektrischer Felder auf das an dem Sensorelement 3 abgegriffene Messignal hängt bei differentieller Spannungsmessung mit kapazitiven Sensoren maßgeblich vom Anpressdruck des Patienten P an das Sensorelement 3 ab. Dieser Druck verändert sich bei der Atmung durch die Gewichtsverlagerung des Körpers des Patienten P. Prinzipiell würde eine auf eine solche Veränderung der elektrischen Felder abstellende Messung einen Rückschluss auf die Atmungsaktivität des Patienten erlauben. Eine Schwierigkeit liegt hier allerdings darin, dass sich die elektrischen Felder auch aus anderen Gründen verändern können und es ad hoc schwer möglich ist, eine Aussage zu treffen, ob eine entsprechende Messgröße auf einer Atembewegung des Patienten P oder anderen extrinsischen oder intrinsischen Effekten des Messsystems 100 beruht. So können durch die Atembewegung induzierte Feldeffekte durch Veränderungen von außen aufgeprägter Feldgrößen maskiert bzw. überlagert werden. Gemäß der vorliegenden Ausführungsform ist daher vorgesehen, die Veränderung der elektrischen Felder auf einzelnen Sensorelementen 3 in ein Verhältnis zu einem Referenzsignal RS zu setzen, sodass auf die kapazitive Kopplung rückgeschlossen werden kann. Als in einer elektrisch betriebenen Umgebung immer verfügbare Signale bieten sich die elektrischen Felder der Netz-Spannungsversorgung bei 50Hz bzw. 60 Hz und deren harmonischen Frequenzen als Referenzsignal RS an. Es ist dann nicht nötig ein eigenes Testsignal aufzuprägen.

Das Referenzsignal RS kann durch eine Referenzsignal-Erfassungsvorrichtung 50 zugeführt werden. Die Referenzsignal-Erfassungsvorrichtung 50 kann z.B. über eine Messanordnung zur Messung eines auf der Schirmung S der kapazitiven Sensoren 3, 4 fließenden Schirmungsstroms umfassen, welcher Schirmungsstrom zwischen der Schirmung S und einem an die einzelnen Sensoren 3 angebundenen GND-Potential fließt. Es kann sowohl jede Schirmung jedes Sensorelements 3 einzeln gemessen werden, als auch ein Schirmungsstrom über einen gemeinsamen Messpunkt, der mit GND verbunden ist, gemessen werden. Es kann auch die Schirmung der Kabelanbindung 6a, 6b der Sensorelemente 3 an die Verstärkerschaltungen der kapazitiven Sensorelemente 3 für die Messung genutzt werden. Außerdem kann der Messpunkt auch zwischen den Verstärkerschaltungen und dem sich diesen anschließenden Einheiten des Messsystems liegen. Ferner kann die Referenzsignal-Erfassungsvorrichtung 50 eine Treiberschaltung für das rechte Bein umfassen. Mit einer solchen Treiberschaltung für das rechte Bein lässt sich eine auf den Patienten direkt wirkende Störung aufgrund eines elektrischen Feldes durch Messen eines sogenannten RLD-Stroms ermitteln und zur Bestimmung des Referenzsignals RS mit einbeziehen. Zusätzlich oder alternativ zu den Vorgenannten kann die Referenzsignal-Erfassungsvorrichtung 50 auch eine Erdstrommesseinheit umfassen, welche dazu eingerichtet ist, einen Strom (Erdstrom), der von der Patientenseite in Richtung der Messsystemseite fließt, zu messen. Der Messpunkt kann zwischen den GND-Punkten der Messseite und dem GND-Punkt der weiterverarbeitenden Schaltung des Messsystems liegen. Der GND-Punkt der weiterverarbeitenden Schaltung kann Erde sein; die Messseite darf und kann für diese Anwendung nicht auf dem Erdpotential liegen.

Die gemessenen Ströme, also der RLD-Strom, der Schirmstrom und der Erdstrom, welche jeweils mit unterschiedlichen Anteilen des Messsignals MS korreliert sind, werden entweder einzeln oder in Kombination als Referenzsignal RS für eine Analyse des Messsignals MS verwendet, um Informationen über eine lokale kapazitive Kopplung und damit eine Atembewegung des Patienten P zu erhalten. Dazu werden aus dem Referenzsignal RS und den Messsignalen MS zunächst die relevanten Frequenzanteile bei 50 Hz bzw. 60 Hz. und den Harmonischen davon extrahiert. Hierfür kann der Vorfilter 34f über ein geeignetes Filtermodul, etwa in Form eines Kammfilters, verfügen. Die so vorbearbeiteten Signale werden dann in ein Komparator-Signalverarbeitungsmodul SVM4 oder Komparatormodul SVM4 eingegeben. Das Komparator-Signalverarbeitungsmodul SVM4 ist dazu ausgebildet, basierend auf den ggf. vorverarbeiteten Messsignalen MS und dem Referenzsignal RS korrigierte Messsignale als bioelektrische Signale S4 zu bestimmen. Mit anderen Worten ist das Komparatormodul SVM4 dazu ausgebildet, die Messsignale MS auf Basis des Referenzsignals RS zu filtern und dadurch den Beitrag von Feldveränderungen, die nicht aus einer lokalen Veränderung des Anpressdrucks des Patienten P an das Sensorelement 3 herrühren, aus den Messsignalen MS herauszurechnen.

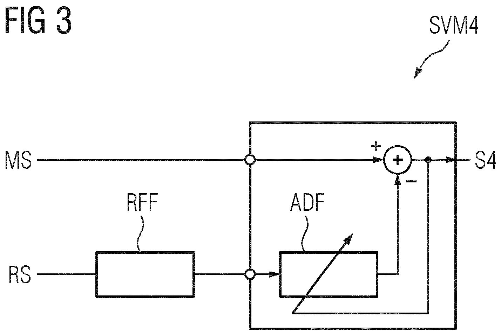

In Figur 3 ist beispielhaft eine mögliche Ausgestaltung des Komparatormoduls SVM4 gezeigt. Das Komparatormodul SVM4 umfasst einen adaptiven Filter ADF sowie einen Referenz-Formungsfilter RFF. Das Komparatormodul SVM4 ist insbesondere dazu eingerichtet, ein korrigiertes Messsignal als bioelektrisches Signal S4 auf Basis eines digitalisierten Messsignals MS und eines digitalisierten Referenzsignals RS zu erzeugen. Der adaptive Filter ADF ist zur Schätzung einer Übertragungsfunktion eingerichtet, die eine Beziehung zwischen dem gemessenen Referenzsignal RS und der auf das Messsignal MS eingekoppelten Modulation durch eine Atmungsbewegung angibt. Mit Hilfe der Übertragungsfunktion erzeugt der adaptive Filter ADF ein geschätztes Subtraktionssignal, welches von dem Messsignal MS subtrahiert werden kann. Weiterhin weist das Komparatormodul SVM4 die Eigenschaft auf, ihre Übertragungsfunktion und damit das geschätzte Subtraktionssignal, und ihre Frequenz im Betrieb selbständig ändern zu können. Dabei wird in Abhängigkeit von einem Ausgangssignal des Kompensationsmoduls SVM4 ein Fehlersignal erzeugt, und die Koeffizienten der von adaptiven Filtereinheit ADF erzeugten und verwendeten Übertragungsfunktion werden in Abhängigkeit von dem Fehlersignal derart geändert, dass das Fehlersignal minimiert wird. Das Komparatormodul SVM4 gibt schließlich ein bioelektrisches Signal S4 aus, das im durch den Kammfilter ausgewählten Frequenzbereich (50 Hz bzw. 60 Hz und deren Harmonische) von Störeffekten durch elektrische Felder befreit ist und so einen Rückschluss auf die kapazitive Kopplung ermöglicht.

Weiterhin umfasst das Komparatormodul SVM4 einen Referenz-Formungsfilter RFF. Dieser Referenz-Formungsfilter RFF ist vorteilhaft, da die gemessenen Referenzsignale RS hinsichtlich ihres Frequenzgangs und ihrer Phase stark von den Messströmen abweichen können und daher sonst, falls es überhaupt möglich ist, nur unter großem Aufwand durch einen adaptiven Filter subtrahiert werden können. Der Referenz-Formungsfilter RFF ist beispielsweise als Tiefpassfilter ausgebildet, der bereits ab wenigen Hz einen Dämpfungseffekt aufweist und bei 20 bis 30 Hz eine Dämpfung von 25 bis 30 dB erreicht. Der Referenz-Formungsfilter RFF kann bevorzugt digital realisiert werden, d.h. als virtueller Filter in Form einer Software ausgebildet sein, er kann aber auch als Hardware realisiert sein.

Die bioelektrischen Signale S1, S2, S3, S4 und die damit verbundenen Ausgestaltungen der Signalverarbeitungsvorrichtung 34 sind als beispielhaft zu verstehen. So kann die Signalverarbeitungsvorrichtung 34 dazu ausgebildet sein, lediglich eines der bioelektrischen Signale S1, S2, S3, S4 bereitzustellen. Alternativ kann die Signalverarbeitungsvorrichtung 34 dazu ausgebildet sein, alle genannten bioelektrischen Signale S1, S2, S3, S4 oder eine beliebige Teilmenge daraus bereitzustellen. Zudem kann die Signalverarbeitungsvorrichtung 34 dazu ausgebildet sein, weitere bzw. alternative bioelektrische Signale bereitzustellen, die von den beispielhaft genannten bioelektrischen Signale S1, S2, S3, S4 abweichen.

Die durch die Signalverarbeitungsvorrichtung 34 ermittelten bioelektrischen Signale S1, S2, S3, S4 werden in die Recheneinheit 35 eingegeben. Die Recheneinheit 35 ist dazu ausgebildet, basierend auf einzelnen bioelektrischen Signalen S1, S2, S3, S4 oder Kombinationen der eingegebenen bioelektrischen Signale S1, S2, S3, S4 eine Atmungsinformation A, Alok zu extrahieren bzw. abzuleiten. Insbesondere kann die Recheneinheit 35 dazu ausgebildet sein, entsprechende Computerprogrammprodukte oder Computerprogrammsegmente zur Ausführung zu bringen, welche zur Bereitstellung der Atmungsinformation A, Alok basierend auf den zur Verfügung stehenden Eingangsgrößen ausgebildet ist. Gemäß einigen Ausführungsformen kann die Recheneinheit 35 eine modellbasierte Berechnung, z.B. basierend auf einem entsprechend ausgebildeten Kalmanfilter, implementieren. Gemäß andern Ausführungsformen kann die Recheneinheit 35 eine trainierte Funktion TF zur Anwendung bringen, die mit Methoden des maschinellen Lernens auf diese Aufgabe angepasst wurde.

Ferner kann das differentielle Spannungsmesssystem 100 eine Trigger-Vorrichtung 36 umfassen. Diese kann dazu ausgebildet sein, aus der Atmungsinformation A, Alok Atmungszyklen des Patienten P zu erkennen, um daraus Steuersignale umfassend eine Trigger- bzw. Startzeitpunktinformation für eine medizinische Bildgebungsanlage B zu generieren. Die Trigger-Vorrichtung 36 kann ferner dazu ausgebildet sein, aus den bioelektrischen Signalen S1, S2, S3, S4 einen Herzschlag des Patienten P zu erkennen, um daraus Steuersignale umfassend eine Trigger- bzw. Startzeitpunktinformation für eine medizinische Bildgebungsanlage B zu generieren. Basierend auf den Steuersignalen der Trigger-Vorrichtung 35 kann eine Bildgebungsanlage B dann z.B. Zeitpunkte für eine Bilddatenerfassung ermitteln.

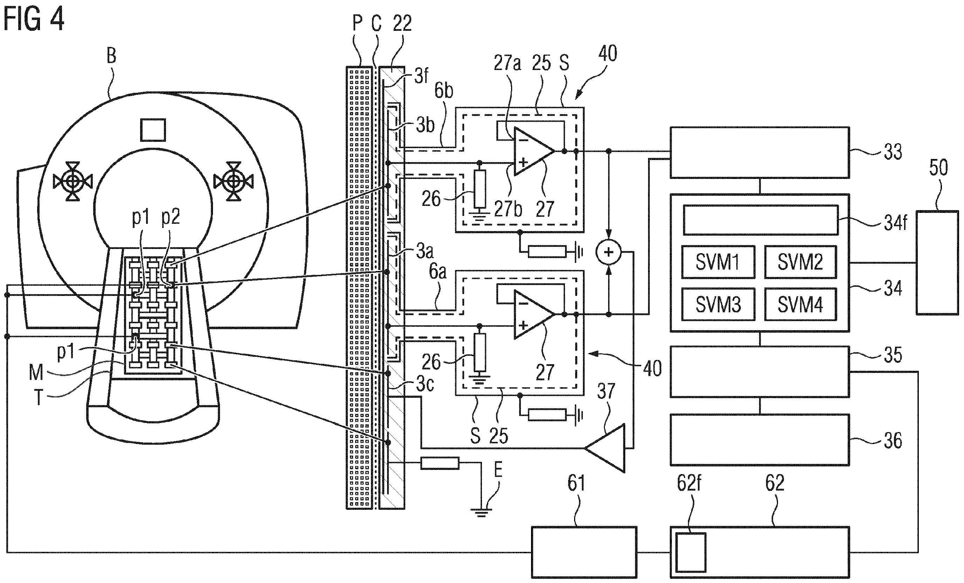

In Figur 4 ist eine weitere beispielhafte Ausführungsform des erfindungsgemäßen Spannungsmesssystems 100 schematisch dargestellt. Gleiche Bezugszeichen wie in den vorherigen Ausführungsformen bezeichnen dabei gleiche oder funktionsgleiche Komponenten.

Die in Figur 4 gezeigte Ausführungsform unterscheidet sich von den vorherigen Ausführungsformen darin, dass die Patientenmatte M zusätzlich Drucksensoren p1, p2 aufweist, die ein lokales Drucksignal DS am Sensorort erfassen und der Verarbeitung als zusätzliche Informationsquelle bereitstellen. Dem liegt die Überlegung zugrunde, dass sich das Gewicht des Patienten P bei der Atmung verschiebt, wodurch die Drucksensoren p1, p2 angeregt werden. Sind mehrere Drucksensoren p an verschiedenen Stellen in der Patientenmatte M enthalten, werden die Drucksensoren p1, p2 je nach Anordnungsposition in der Patientenmatte M unterschiedlich stark angeregt und aus den Drucksignalen DS ergibt sich ein lokal aufgelöstes Bild der Druckbelastung der Patientenmatte M durch den Patienten P. Die Drucksensoren p1, p2 können an für eine Atmungserkennung geeigneten Position angebracht sein. Eine solche Position ist z.B. in der Mitte der Patientenmatte M unter der Wirbelsäule des Patienten P, wo der Patient P nicht dauerhaft aufliegt und sich durch die Volumenänderung bei der Atmung ein unterschiedlicher Abstand zwischen Patient P und Patientenmatte M ergibt. Ein hier gegenüber dem Rest der Patientenmatte M hervorstehend eingebauter Drucksensor p1, p2 würde durch diese Abstandsänderung stark angeregt.

Die mit den Drucksensoren p1, p2 ermittelten Drucksignale DS werden über Signalmesskabel K über geeignete Anschlüsse, meist Steckverbindungen, in eine sekundäre Signalverarbeitungsvorrichtung 62 eingegeben. Die erste Drucksensoren p einschließlich der Signalmesskabel K bilden dabei einen Teil eines sekundären Nutzsignalpfads, mit der die Drucksignale DS der Drucksensoren p1, p2 erfasst werden können. Die Druckmesssignale DS können dabei vor der Eingabe in die sekundäre Signalverarbeitungsvorrichtung 62 durch einen AD-Wandler (nicht gezeigt) digitalisiert werden. Die sekundäre Signalverarbeitungsvorrichtung 62 ist dazu ausgebildet, aus den Drucksignalen DS eine Information zu extrahieren, die eine, insbesondere lokale, Druckbelastung durch den Patienten P indiziert. Der sekundären Signalverarbeitungsvorrichtung 62 kann optional eine Schaltvorrichtung 61 vorgeschaltet sein, die ähnlich der Switch Matrix 33 auszuwählen kann, welche der Drucksensoren p1, p2 für eine weitere Signalverarbeitung verwendet werden. Ferner kann die sekundäre Signalverarbeitungsvorrichtung 62 einen Vorfilter 62f aufweisen, der die eingehenden Drucksignale DS nach geeigneten Frequenzen filtert. Der Frequenzbereich kann dabei zwischen 0,05 Hz und 5 Hz liegen. Der Vorfilter 62f kann durch physische, d.h. Hardwarebasierte, Module und/oder virtuelle, insbesondere software-basierte, Module realisiert sein.

In der sekundären Signalverarbeitungsvorrichtung 62 werden die Drucksignale DS zu einem Signal SN verarbeitet, das eine Druckbelastung der Patientenmatte M durch den Patienten P angibt (im Folgenden auch Druckbelastungssignal genannt). Dieses Signal SN wird dann in die Recheneinheit 35 eingegeben, die entsprechend dazu ausgebildet ist, das Signal SN bei der Atmungserkennung, d.h. der Bestimmung der Atmungsinformation A, Alok zusätzlich zu berücksichtigen. Gemäß einigen Ausführungsformen kann die Recheneinheit 35 auch derart ausgebildet sein, dass sie die Atmungsinformation A, Alok allein basierend auf dem Signal SN, d.h. ohne Berücksichtigung der bioelektrischen Signale S1, S2, S3, S4 bestimmt.

Gemäß Ausführungsformen der Erfindung sind zwei verschiedene Arten Drucksensoren p1, p2 denkbar. Zum einen können in die Patientenmatte M separate Drucksensoren p1 umfasst sein, die unabhängig von den Sensorelementen 3 zur differentiellen Spannungsmessung ausgeführt sind. Hierfür können aus dem Stand der Technik bekannte Drucksensoren verwendet werden.

Die Kombination der Druckmessung mit der Ermittlung der bioelektrischen Signale S1, S2, S3, S4 eröffnet darüber hinaus die Möglichkeit, Drucksensoren als integrierte Drucksensoren p2 in den Sensoraufbau der kapazitiven Sensorelemente 3 zu integrieren. Dabei können die integrierten Drucksensoren p2 insbesondere in eine mechanische Aufhängung 10 für die Sensorelektrode 3a integriert sein.

Eine Ausführungsform für ein solche mechanische Aufhängung 10 ist in Figur 5 dargestellt. Die mechanische Aufhängung 10 ist zumindest teilweise, also zumindest in Teilbereichen, kompressibel ausgebildet, d.h. sie lässt sich komprimieren. Die mechanische Aufhängung 10 ist an einem Untergrund U des Sensorelement-Aufbaus befestigt und stützt die Sensorelektrode 3 gegen den Untergrund U ab. Der Untergrund U ist bspw. durch die Unterseite/die Auflagefläche einer kapazitiven Patientenmatte M ausgebildet. Als direktes Stützelement für die Sensorelektrode 3 umfasst die mechanische Aufhängung 10 eine Stützstruktur 5. Die Stützstruktur 5 ist unterhalb der Sensorelektrode angeordnet, sie weist eine mindestens der Grundfläche der Sensorelektrode 3 entsprechende Grundfläche auf, sodass die Sensorelektrode 3 vollständig auf der Stützstruktur 5 aufliegt. Die mechanische Aufhängung 10 umfasst als weiteren Bestandteil eine Trägerstruktur 7. Diese ist unterhalb von Stützstruktur 5 und oberhalb des Untergrundes U angeordnet. Die Stützstruktur 5 ist insbesondere aus kompressiblem Schaumstoff ausgebildet. Die Trägerstruktur 7 bedeckt den Untergrund in dieser Ausführung vollflächig über die Grundfläche des Messsystems 1 hinweg. Die Trägerstruktur 7 ist in dieser Ausführung ebenfalls aus einem kompressiblen Schaumstoff gebildet. Die Trägerstruktur 7 ist dabei insbesondere härter ausgebildet als die Stützstruktur 5. Vorliegend ist die Trägerstruktur 7 um 25% härter als die Stützstruktur 5 ausgebildet. Insbesondere in Ausgestaltungen, in denen das Sensorelement 3 vielfach bspw. in einer kapazitiven Patientenmatte integriert ist, erlaubt die Trägerstruktur einen Ausgleich eines unterschiedlichen Höhenniveaus der Patientenoberfläche. Bspw. kann mit der Trägerstruktur 7 ein Hohlkreuz-Verlauf ausgeglichen werden, sodass trotz des variablen Höhenniveaus alle einzelnen Messsysteme 1 der Patientenmatte M ein Messsignal MS liefern können. Bei Hohlkreuz würde die Trägerstruktur 7 kaum komprimiert werden, bei geradem Rücken des Patienten P entsprechend stärker.

Die Grundfläche der Stützstruktur 5 ist hier beispielhaft quadratisch mit den Maßen 3 cm x 3 cm entsprechend der Grundfläche der Sensorelektrode 3a. Die Höhe der Stützstruktur 5 beläuft sich beispielhaft auf 8 cm. Die Stützstruktur besteht vorliegend aus einem viskoelastischen Schaumstoff mit hoher Energieabsorption, insbesondere dem Schaumstoff GV 50/30. Dieser zeichnet sich dadurch aus, dass er für kleine Kompressionsänderungen eine im Wesentlichen konstante Gegenkraft erzeugt.

Um einen über die Grundfläche des Messsystems 1 besonders gleichmäßigen Krafteintrag in die Trägerstruktur 7 zu erreichen, umfasst in dieser Ausführung die mechanische Aufhängung 10 optional eine Zwischenschicht 9. Diese verbindet die Trägerstruktur 7 mit der Stützstruktur 5. Das heißt, sie ist oberhalb der Trägerstruktur 7, ebenfalls vollflächig zumindest über die Grundfläche des Messsystems 1, hinweg ausgebildet. Die optionale Zwischenschicht 9 sorgt insbesondere eine Verteilung von bewegungsbedingt lokal sehr begrenzt wirkenden Kraftspitzen auf weitere Bereiche der Trägerstruktur, wodurch ein Neigen bzw. Verkippen von Stützstruktur 5 bzw. Sensorelektrode 3a minimiert und damit die Sensorfläche immer im Wesentlichen planparallel zur Patientenoberfläche ausgerichtet bleibt, um einen optimalen Signalabgriff zu gewährleisten.

Zur Bereitstellung der Drucksignale DS wird Trägerstruktur 7 zum Messelement, bzw. integrierten Drucksensor p2, aufgerüstet. Dazu kann ein Schaumstoff der Trägerstruktur 7 zum Beispiel so leitfähig ausgeprägt sein, dass er bei Kompression seine Leitfähigkeit verändert. Durch eine kontinuierliche Messung der aktuellen Volumenimpedanz kann so die aktuelle Kompression erfasst werden. Diese Messung kann durch zwei leitfähige Schichten, je eine direkt ober- und eine unterhalb der der Trägerstruktur 7, erfolgen, welche von dem sekundären Nutzsignalpfad mit einem konstanten Strom beaufschlagt werden, und der sekundären Nutzsignalpfad die dadurch abfallende Spannung erfasst und an die sekundäre Signalverarbeitungsvorrichtung 62 als Druckmesssignal DS weiterleitet.

Gemäß einer in Figur 6 gezeigten alternativen Ausführungsform umfasst die mechanische Aufhängung 10 ferner eine Rahmenstruktur 4. Die Rahmenstruktur 4 umgibt die Stützstruktur 5 zumindest teilweise. In dieser Ausführung ist die Rahmenstruktur 4 an zwei Seiten der Stützstruktur 5 vorgesehen. In alternativen Ausführungen kann die Rahmenstruktur 5 eine um die Stützstruktur 5 beschlossene Struktur sein. Die Stützstruktur 5 ist höher als die Rahmenstruktur 4 ausgebildet. Sie erstreckt sich also in unbelastetem Zustand weiter vom Untergrund U in Richtung Patient P als die Rahmenstruktur 4.

Die Rahmenstruktur 4 kann aus einem kompressiblen Material, insbesondere einem Schaumstoff (fördert den Patientenkomfort) gebildet sein. Sie kann aber auch aus einem inkompressiblen Material, bspw. einem Kunststoff oder Holz bestehen. In jedem Fall weist die Stützstruktur 5 eine geringere Härte als die Rahmenstruktur 4 auf. Die Stützstruktur 5 weist folglich eine höhere Kompressibilität als die Rahmenstruktur 4 auf. Die Rahmenstruktur 4 muss in jedem Fall so fest gewählt werden, dass sie die Stützstruktur 5 konstant und längerfristig im Wesentlichen auf der Höhe der Rahmenstruktur 4 halten kann. Durch eine Kraftkomponente FKomp der Gewichtskraft FBody des Patienten P wird nun die Stützstruktur 5 im Wesentlichen auf die Höhe der Rahmenstruktur 4 zusammengepresst. Der überwiegende/restliche Teil der Gewichtskraft (FBody - FKomp) wird auf die weniger oder nicht kompressible Rahmenstruktur 4 übertragen. Die auf die Sensorelektrode 3a bzw. die Stützstruktur 5 wirkende Kraftkomponente FKomp verursacht eine durch die Stützstruktur 5 erzeugte Gegenkraft FFoam in Richtung Patient P, die betragsmäßig der Kraftkomponente FKomp entspricht, also FKomp = - FFoam. Diese Gegenkraft FFoam bleibt nun auch bei Patientenbewegung, bspw. durch Gewichtsverlagerung, oder Herzschlag konstant, da eine maximale Kompression der Stützstruktur 5 durch die Rahmenstruktur 4 vorgegeben ist. Eine Variation der wirkenden Gewichtskraft FBody wirkt sich erfindungsgemäß nur auf die Rahmenstruktur 4, jedoch nicht mehr auf die Stützstruktur 5 aus. Dies gewährt einerseits eine Ableitung der Druckbelastung durch den Patienten an die Trägerstruktur 7, in welcher der integrierte Drucksensor p2 angeordnet ist (s.o.). Andererseits wird so eine Qualität der der Messung der bioelektrischen Signale S1, S2, S3, S4 verbessert, da hierdurch Störungen auf das Messsignal MS eliminiert oder zumindest vermindert werden.

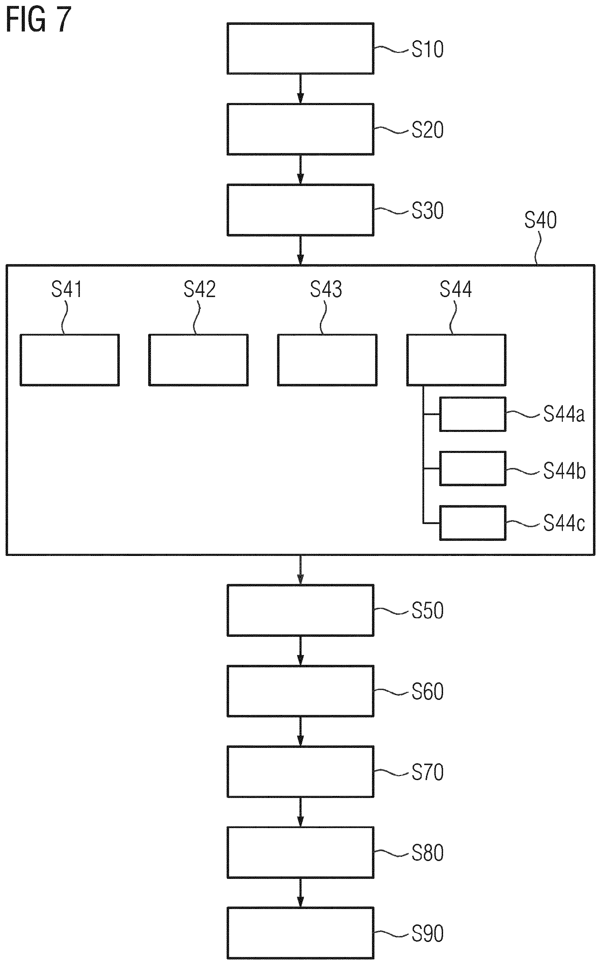

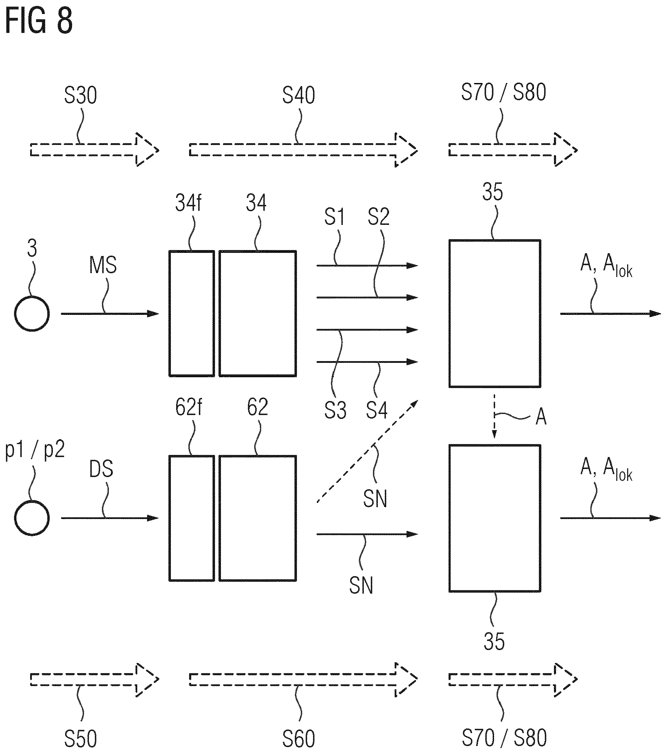

In Figur 7 ist ein schematisches Ablaufdiagramm eines Verfahrens zur Bereitstellung einer eine Atmungsaktivität eines Patienten P dargestellt. Die Reihenfolge der Verfahrensschritte ist weder durch die dargestellte Abfolge noch durch die gewählte Nummerierung beschränkt. So kann die Reihenfolge der Schritte ggf. vertauscht und einzelne Schritte können weggelassen werden. Außerdem können ein oder mehrere Schritte, insbesondere eine Sequenz von Schritten, und optional das gesamte Verfahren wiederholt ausgeführt werden. In Figur 8 sind mit dem in Figur 7 gezeigten Verfahren assoziierte Datenströme exemplarisch dargestellt.

In einem ersten Schritt S10 wird ein differentielles Spannungsmesssystem nach einem der hierin beschriebenen Ausführungsformen bereitgestellt. In einem Schritt S20 wird ein Patient P auf der Patientenmatte M angeordnet.

Schritt S30 ist auf die Erfassung wenigstens eines Messsignals MS mit wenigstens einer kapazitiven Sensorelektrode 3a, 3b, 3c gerichtet. Schritt S30 kann dabei eine geeignete Vorverarbeitung der Messsignale MS wie etwa eine Umwandlung analoger Messsignale MS in digitale Messsignale MS oder eine Auswahl geeigneter Messsignale MS aus einer Vielzahl an Messsignalen MS von verschiedenen Sensorelektroden 3a, 3b, 3c umfassen. Schritt S30 erfolgt bevorzugt mittels der Signalmessschaltungen 40 bzw. ggf. der Schaltmatrix 33 und ggf. eines in die Messstrecke integrierten AD-Wandlers.

In Schritt S40 erfolgt eine Bestimmung eines oder mehrerer bioelektrischer Signale S1, S2, S3, S4 basierend auf den Messsignalen MS insbesondere mit Mitteln der digitalen Signalverarbeitung. Dazu weist Schritt S40 die optionalen Unterschritte S41-S44 auf. Schritt S40 kann einen oder mehrere der Unterschritte S41-S44 umfassen. In Schritt S41 wird aus den in Schritt S30 erfassten und ggf. vorverarbeiteten Messsignalen MS ein bioelektrisches Signal S1 bestimmt, das einen Beat-to-Beat Herzschlag des Patienten P umfasst. Dazu können Messsignale MS geeignet gefiltert werden, um relevante Signalanteile zu erhalten, welche z.B. im Bereich von 1 Hz bis 40 Hz liegen können. In Schritt S42 wird aus den in Schritt S30 erfassten und ggf. vorverarbeiteten Messsignalen MS (bevorzugt aus einer Vielzahl an Messsignalen MS) ein bioelektrisches Signal S2 bestimmt, das einen EKG-vektor des Patienten P umfasst.

Dabei kann insbesondere ausgenutzt werden, dass bei der Verwendung mehrerer unterschiedlich in der Patientenmatte M angeordneter Sensoren 3 eine ortsaufgelöste Messung der Messsignale MS erfolgt, was die Bestimmung eines EKG-Raumvektors ermöglicht. Auch hier liegen relevante Signalanteile im Bereich 1 Hz bis 40 Hz und ein entsprechender Filterschritt der Messsignale MS kann in Schritt S42 optional inkludiert sein. In Schritt S43 wird aus den in Schritt S30 erfassten und ggf. vorverarbeiteten Messsignalen MS ein bioelektrisches Signal S2 bestimmt, das eine Muskelaktivität des Patienten P umfasst. Relevante Signalanteile liegen dabei im Bereich von 10 Hz bis 100 Hz. Ein entsprechender Filterschritt ist in Schritt S43 optional enthalten.

In Schritt S44 wird aus den in Schritt S30 erfassten und ggf. vorverarbeiteten Messsignalen MS ein bioelektrisches Signal S4 bestimmt, das eine kapazitive Kopplung des Patienten P mit der jeweiligen Sensorelektrode 3a, 3b, 3c indiziert und so einen Rückschluss auf die Atmungsaktivität des Patienten P zulässt. Dazu werden die Messsignale MS mit einem Referenzsignal RS gefiltert, um Schwankungen in den elektrischen Feldern zu kompensieren. In einem Teilschritt S44a erfolgt entsprechend eine Erfassung des Referenzsignals RS mit der Referenzsignal-Erfassungsvorrichtung 50. In einem weiteren Teilschritt S44b werden aus dem Referenzsignal RS und den Messsignalen MS zunächst die relevanten Frequenzanteile bei 50 Hz bzw. 60 Hz. und den Harmonischen davon extrahiert. Hierfür kann insbesondere ein Kammfilter verwendet werden. In einem weiteren Teilschritt S44c erfolgt die Bestimmung eines korrigierten Messignals als bioelektrisches Signal S4 durch Filtern der Messsignale MS auf Basis des Referenzsignals RS.

Schritt S40 einschließlich möglicher Teilschritte erfolgt mittels der Signalverarbeitungsvorrichtung 34 und insbesondere unter Verwendung der darin applizierten Module SVM1, SVM2, SVM3, SVM4 und 34f.

Schritt S50 ist auf die Erfassung wenigstens eines Drucksignals DS mit den Drucksensoren p1 bzw. p2 gerichtet. Hierfür kann mit den sekundären Nutzsignalpfaden ein Signal abgegriffen werden, das die lokale Druckbelastung an den Drucksensoren p1 und p2 indiziert. In Schritt S60 wird basierend auf diesen Drucksignalen DS ein Signal SN ermittelt, das eine, insbesondere lokale, d.h. ortsaufgelöste, Druckbelastung der Patientenmatte M durch den Patienten P angibt. Diese Bestimmung kann mittels der sekundären Signalverarbeitungsvorrichtung 62 erfolgen.

In Schritt S70 erfolgt die Bestimmung der Ein- und Ausatemzüge des Patienten P als eine die Atmung des Patienten P indizierende Information A basierend auf einem oder mehreren der bioelektrischen Signale S1, S2, S4, S4 und/oder dem Druckbelastungssignal SN. Insbesondere kann Schritt S70 ein Anwenden eines Kalman-Filters und oder einer trainierten Funktion TF auf die Signale S1, S2, S3, S4 und/oder SN umfassen. Schritt S70 kann insbesondere durch eine entsprechend ausgebildete Recheneinheit 35 erfolgen.

In dem optionalen Schritt S80 können die in Schritt S70 bestimmten Ein- und Ausatemzüge verwendet werden, um unter zusätzlicher Berücksichtigung des ortsaufgelösten Druckbelastungssignals SN als eine die Atmung des Patienten P indizierende Information Alok eine lokale Atmungskomponente zu bestimmen, welche angibt, wie sich die Ein- und Ausatemzüge auf wenigstens ein Körperteil des Patienten P auswirken. Optional können in Schritt S80 auch ein oder mehrere bioelektrische Signale S1, S2, S3, S4 nochmals gesondert berücksichtigt werden - insbesondere das EKG-Vektor-Signal S2 oder das eine (lokale) kapazitive Kopplung indizierende Signal S4, da diese ebenfalls lokal aufgelöste Informationen enthalten. Insbesondere kann Schritt S80 ein Anwenden eines Kalman-Filters und oder einer trainierten Funktion TF auf die in Schritt S70 bestimmten Ein- und Ausatemzüge des Patienten P sowie die Signale S1, S2, S3, S4 und/oder SN umfassen. Schritt S80 kann insbesondere durch eine entsprechend ausgebildete Recheneinheit 35 erfolgen.

Schritt S90 ist schließlich auf ein Bereitstellen der bestimmten Ein- und Ausatemzüge bzw. der lokalen Atmungskomponente insbesondere an eine medizinische Bildgebungsanlage B gerichtet, in welchem bildgebenden System diese Atmungsinformation A, Alok verwendet werden kann, um eine Erfassung medizinischer Bilddaten zu optimieren und insbesondere an die Atmung des Patienten P anzupassen.

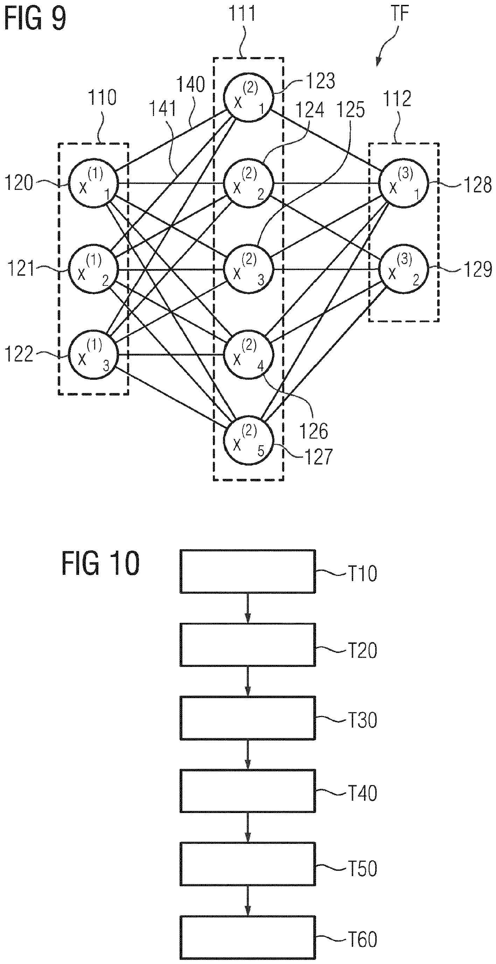

In **Figur 9** ist eine trainierte Funktion TF schematisch vereinfacht dargestellt, welche zur Bestimmung einer Atmungsinformation A, $A_{lok}$ aus den bioelektrischen Signalen S1, S2, S3, S4 und/oder dem Druckbelastungssignal SN geeignet ist bzw. hierfür trainiert werden kann. In dem gezeigten Ausführungsbeispiel ist die trainierte Funktion TF als neuronales Netz ausgebildet. Das neuronale Netz kann auch als künstliches neuronales Netz, künstliches neuronales Netzwerk oder neuronales Netzwerk bezeichnet werden.

Das neuronale Netz 100 umfasst Knoten 120,..., 129 und Kanten 140,141, wobei jede Kante 140,141 eine gerichtete Verbindung von einem ersten Knoten 120,..,129 zu einem zweiten Knoten 120,..., 129 ist. Im Allgemeinen sind der erste Knoten 120,...,129 und der zweite Knoten 120,...,129 unterschiedliche Knoten, es ist auch möglich, dass der erste Knoten 120,...,129 und der zweite Knoten 120,...,129 identisch sind. Eine Kante 140, 141 von einem ersten Knoten 120,...,129 zu einem zweiten Knoten 120,...,129 kann auch als eingehende Kante für den zweiten Knoten und als ausgehende Kante für den ersten Knoten 120,...,129 bezeichnet werden.

Das neuronale Netz 100 antwortet auf Eingabewerte $x^{(1)}_1$, $x^{(1)}_2$, $x^{(1)}_3$ zu einer Vielzahl von Eingangsknoten 120, 121, 122 der Eingangsschicht 110. Die Eingabewerte $x^{(1)}_1$, $x^{(1)}_2$, $x^{(1)}_3$ werden angewendet, um eine oder eine Vielzahl von Ausgaben $x^{(3)}_1$, $x^{(3)}_2$ zu erzeugen. Der Knoten 120 ist beispielsweise über eine Kante 140 mit dem Knoten 123 verbunden. Der Knoten 121 ist beispielsweise über die Kante 141 mit dem Knoten 123 verbunden.

Das neuronale Netz 100 lernt in diesem Ausführungsbeispiel, indem es die Gewichtungsfaktoren $w_{i,j}$ (weights) der einzelnen Knoten basierend auf Trainingsdaten anpasst. Mögliche Eingabewerte $x^{(1)}_1$, $x^{(1)}_2$, $x^{(1)}_3$ der Eingangsknoten 120, 121, 122 können beispielsweise die bioelektrischen Signale S1, S2, S3, S4, das Druckbelastungssignal SN und/oder eine Information über Ein- und Ausatemzüge des Patienten P sein.

Das neuronale Netz 100 gewichtet die Eingabewerte der Eingangsschicht 110 basierend auf dem Lernprozess. Die Ausgabewerte der Ausgangsschicht 112 des neuronalen Netzes 100 entsprechen bevorzugt einer die Atmung des Patienten P indizierenden Information A, Alok, wie etwa den Ein- und Ausatemzügen des Patienten oder der lokalen Atemkomponente des Patienten. Die Ausgabe kann über einen einzelnen oder eine Vielzahl von Ausgabeknoten $x^{(3)}_1$, $x^{(3)}_2$ in der Ausgabeschicht 112 erfolgen.

Das künstliche neuronale Netz 100 umfasst bevorzugt eine versteckte Schicht 111, die eine Vielzahl von Knoten $x^{(2)}_1$, $x^{(2)}_2$, $x^{(2)}_3$ umfasst. Es können mehrere versteckte Schichten vorgesehen sein, wobei eine versteckte Schicht Ausgabewerte einer anderen versteckten Schicht als Eingabewerte verwendet. Die Knoten einer versteckten Schicht 111 verrichten mathematische Operationen. Ein Ausgabewert eines Knotens $x^{(2)}_1$, $x^{(2)}_2$, $x^{(2)}_3$ entspricht dabei einer nicht-linearen Funktion f seiner Eingabewerte $x^{(1)}_1$, $x^{(1)}_2$, $x^{(1)}_3$ und der Gewichtungsfaktoren $w_{i,j}$. Nach dem Erhalt von Eingabewerten $x^{(1)}_1$, $x^{(1)}_2$, $x^{(1)}_3$ führt ein Knoten $x^{(2)}_1$, $x^{(2)}_2$, $x^{(2)}_3$ eine Summierung einer mit den Gewichtungsfaktoren $w_{i,j}$ gewichteten Multiplikation jedes Eingabewerts $x^{(1)}_1$, $x^{(1)}_2$, $x^{(1)}_3$ durch, wie durch folgende Funktion bestimmt:

$$x^{(n+1)}_j = f\left(\sum_i x^{(n)}_i \cdot w^{(m,n)}_{i,j}\right).$$

Der Wichtungsfaktor $w_{i,j}$ kann insbesondere eine reelle Zahl, insbesondere im Intervall von [-1;1] oder [0;1] liegen. Der Wichtungsfaktor $w^{(m,n)}_{i,j}$ bezeichnet das Gewicht der Kante zwischen dem i-ten Knoten einer m-ten Schicht 110,11,112 und einem j-ten Knoten der n-ten Schicht 110,111,112. Insbesondere wird ein Ausgabewert eines Knotens $x^{(2)}_1$, $x^{(2)}_2$, $x^{(2)}_3$ als Funktion f einer Knoten-Aktivierung, beispielsweise eine Sigmoidalfunktion oder eine lineare Rampenfunktion gebildet. Die Ausgabewerte $x^{(2)}_1$, $x^{(2)}_2$, $x^{(2)}_3$ werden an den bzw. die Ausgabeknoten 128,129 übertragen. Erneut wird eine Summierung einer gewichteten Multiplikation jedes Ausgabewertes $x^{(2)}_1$, $x^{(2)}_2$, $x^{(2)}_3$ als Funktion der Knoten-Aktivierung f und damit die Ausgabewerte $x^{(3)}_1$, $x^{(3)}_2$ berechnet.

net.

Das hier gezeigte neuronale Netz TF ist ein Feedforward neuronales Netz, bei dem alle Knoten 111 die Ausgabewerte einer vorherigen Schicht in Form ihrer gewichteten Summe als Eingabewerte verarbeiten. Selbstredend können erfindungsgemäß auch andere neuronale Netztypen zum Einsatz kommen, bspw. Feedback-Netze, bei denen ein Eingabewert eines Knotens gleichzeitig auch sein Ausgabewert sein kann.

Das neuronale Netz TF kann mittels einer Methode des überwachten Lernens trainiert werden, um die Atmungsinformationen A, Alok bereitzustellen. Eine bekannte Vorgehensweise ist die Back-Propagation, die für alle Ausführungsbeispiele der Erfindung angewandt werden kann. Während des Trainings wird das neuronale Netz TF auf Trainingseingabedaten bzw. - werten angewandt und muss entsprechende, vorher bekannte Trainingsausgabedaten bzw. -werte erzeugen. Iterativ werden mittlere quadratische Fehler (mean square error - "MSE") zwischen berechneten und erwarteten Ausgabewerten berechnet und einzelne Gewichtungsfaktoren so lange angepasst, bis die Abweichung zwischen berechneten und erwarteten Ausgabewerten unterhalb einer vorbestimmten Schwelle liegt.

Zur Bereitstellung von Trainingseingabedaten können dabei bioelektrische Signale S1, S2, S3, S4 bzw. Druckbelastungssignale SN von einer auf der Patientenmatte M angeordneten Testperson P im wesentlich wie in Zusammenhang mit den vorherigen Ausführungsformen insbesondere unter Verwendung eines differentiellen Spannungsmesssystems 100 erzeugt werden. Geeignete Trainingsausgabedaten können besonders effizient dadurch erzeugt werden, indem parallel die Atmung der Testperson P aufgezeichnet wird, um den Atmungsinformationen A, Alok, die die trainierte Funktion TF letztlich bereitstellen soll, entsprechende Vergleichsdaten zu sammeln. Dies kann z.B. mit Mitteln der Spirometrie etwa unter Verwendung eines Spirometers erfolgen. So lassen sich mit einem Spirometer z.B. die Ein- und Ausatemzüge des Patienten während der Messung der bioelektrische Signale S1, S2, S3, S4 bzw. der Druckbelastungssignale SN erfassen. Alternativ oder zusätzlich können Trainingsausgabedaten z.B. durch eine Kamera- oder Radar-basierte Erfassung der Atmung der Testperson P erzeugt werden. Insbesondere kann dadurch eine lokale Atemkomponente Alok der Testperson P bestimmt werden. Zusätzlich oder alternativ kann die Testperson P während der Messung der Signale S1, S2, S3, S4, SN mit einem oder mehreren Atemmessgurten versehen werden, welche gleichsam eine Bestimmung der Atmungsinformation erlauben.

Figur 10 zeigt eine beispielhafte Ausgestaltung eines computer-implementierten Verfahrens zur Bereitstellung einer trainierten Funktion TF. Die Reihenfolge der Verfahrensschritte ist weder durch die dargestellte Abfolge noch durch die gewählte Nummerierung beschränkt. So kann die Reihenfolge der Schritte ggf. vertauscht und einzelne Schritte können weggelassen werden. Außerdem können ein oder mehrere Schritte, insbesondere eine Sequenz von Schritten, und optional das gesamte Verfahren wiederholt ausgeführt werden.

In Schritt T10 werden Trainingseingabedaten bereitgestellt, wobei die Trainingseingabedaten insbesondere ein oder mehrere bioelektrische Trainings-Signale S1, S2, S3, S4 und/oder ein Trainings-Druckbelastungssignal SN umfassen, die von einer Testperson P bevorzugt mittels eines differentiellen Spannungsmesssystems 100 bestimmt wurden.

In Schritt T20 werden Trainingsausgabedaten bereitgestellt, wobei die Trainingsausgabedaten mit den Trainingseingabedaten in Verbindung stehen, und insbesondere eine parallel zur Bestimmung der Trainingseingabedaten bestimmte Atmungsinformation A, Alok umfassen. Dabei werden die Trainingsausgabedaten insbesondere von einer Messeinrichtung erfasst, die von dem differentiellen Spannungsmesssystem 100 verschieden ist und insbesondere auf einem unterschiedlichen Messprinzip beruht.

In einem Schritt T30 wird die trainierte Funktion TF auf die Trainingseingabedaten angewandt, um intermediäre Ausgabedaten zu erzeugen. In Schritt T40 werden diese intermediären Ausgabedaten mit den Trainingsausgabedaten verglichen, woraufhin die trainierte Funktion TF in Schritt T50 basierend auf dem Vergleich angepasst wird. Dies kann beispielsweise auf Grundlage eines Kostenfunktionals geschehen, das Abweichungen der Atmungsinformation A, Alok in den intermediärem Ausgabedaten von jener in den Trainingsausgabedaten bestraft. Ein oder mehrere Parameter der trainierten Funktion TF können dann insbesondere so angepasst werden, dass das Kostenfunktional minimiert wird, beispielsweise mittels einer Rückpropagation (ein englischer Fachbegriff ist "back propagation"). Zur Minimierung des Kostenfunktionals wird der Vergleich für verschiedene paarweise Sätze aus Trainingsausgabedaten und Trainingsausgabedaten sowie intermediären Ausgabedaten durchgeführt, bis ein lokales Minimum des Kostenfunktionals erreicht ist und die trainierte Funktion TF zufriedenstellend arbeitet. In Schritt T60 wird die so angepasste trainierte Funktion TF schließlich bereitgestellt.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

**Patentansprüche**

1. Differentielles Spannungsmesssystem (100) zur Bestimmung einer Atmungsaktivität eines Patienten (P), mit:

> einer Anzahl von Signalmessschaltungen (40) mit jeweils einem kapazitiven Sensorelement (3) zur Erfassung eines auf den Patienten (P) bezogenen Messsignals (MS);
> einer Signalverarbeitungsvorrichtung (34) zur Bestimmung wenigstens eines bioelektrischen Signals (S1, S2, S3, S4) aus den Messignalen (MS); und
> einer Recheneinheit (35), die dazu ausgebildet ist, basierend auf dem wenigstens einen bioelektrischen Signal (S1, S2, S3, S4) eine Atmungsinformation (A, A$_{lok}$) zu ermitteln und diese bereitzustellen, welche Atmungsinformation (A, A$_{lok}$) eine Atmungsaktivität des Patienten (P) indiziert.

2. Differentielles Spannungsmesssystem (100) nach Anspruch 1, bei dem:

> die Signalverarbeitungsvorrichtung (34) zur Bestimmung wenigstens zweier verschiedener bioelektrischer Signale (S1, S2, S3, S4) aus den Messignalen (MS) ausgebildet ist; und
> die Recheneinheit (35) dazu ausgebildet ist, die wenigstens zwei verschiedenen bioelektrischen Signale (S1, S2, S3, S4) jeweils bei der Ermittlung der Atmungsinformation (A, A$_{lok}$) zu berücksichtigen.

3. Differentielles Spannungsmesssystem (100) nach einem der vorhergehenden Ansprüche, bei dem das wenigstens eine bioelektrische Signal (S1, S2, S3, S4) umfasst: eine Beat-to-Beat Herzrate (S1) des Patienten (P), einen EKG Vektor (S2) des Patienten (P), und/oder ein Muskelaktivitätssignal (S3) des Patienten (P).

4. Differentielles Spannungsmesssystem (100) nach einem der vorhergehenden Ansprüche, bei dem das wenigstens eine bioelektrische Signal (S4) eine kapazitive Kopplung zwischen Sensorelement (3) und Patient (P) indiziert; und die Signalverarbeitungsvorrichtung (34) aufweist:

> eine Referenzsignalerfassungsvorrichtung (50), welche dazu eingerichtet ist, ein Referenzsignal (RS) zu erfassen; und
> eine Komparatormodul (SVM4), welches dazu eingerichtet ist, basierend auf dem Referenzsignal (RS) ein korrigiertes Messsignal zu ermitteln und basierend auf dem korrigierten Messsignal als bioelektrisches Signal (S4) ein Signal bereitzustellen, aus dem eine kapazitive Kopplung zwischen wenigstens einem der Sensorelemente (3) und dem Patienten (P) ableitbar ist.

5. Differentielles Spannungsmesssystem (100) nach Anspruch 4, bei dem
   das Komparatormodul (SVM4) umfasst:

   > einen Referenz-Formungsfilter (RFF), welcher dazu eingerichtet ist, das Referenzsignal (RS) zu filtern; und
   > eine adaptive Filtereinheit (ADF), welche dazu eingerichtet ist, das Messsignal (MS) auf Basis des gefilterten Referenzsignals zu filtern und so das korrigierte Messsignal zu ermitteln.

6. Differentielles Spannungsmesssystem (100) nach einem der Ansprüche 4 oder 5, bei dem
   die Signalverarbeitungsvorrichtung (34) einen Vorfilter (34f), insbesondere in Form eines Kammfilters, aufweist, der dazu ausgebildet ist, aus dem Messignal (MS) und/oder dem Referenzsignal (RS) Frequenzen von 50Hz, 60Hz und/oder deren Harmonische zu extrahieren.

7. Differentielles Spannungsmesssystem (100) nach einem der vorhergehenden Ansprüche, ferner mit:

   > einer Anzahl drucksensitiver Messelemente (p1, p2) jeweils zur Erfassung eines eine lokale Druckbelastung durch den Patienten (P) auf eine Unterlage (M) indizierenden Drucksignals (DS); und
   > einer sekundären Signalverarbeitungsvorrichtung (62) zur Bestimmung wenigstens eines, insbesondere ortsaufgelösten, Druckbelastungssignals (SN) aus den Drucksignalen (DS); wobei die Recheneinheit (35) ferner dazu ausgebildet ist, die eine Atmungsinformation (A, $A_{lok}$) zusätzlich basierend auf dem Druckbelastungssignal (SN) zu ermitteln und bereitzustellen.

8. Differentielles Spannungsmesssystem (100) nach Anspruch 7, bei dem:

   > wenigstens eines der kapazitiven Sensorelemente (3) eine mechanische Aufhängung (10) aufweist, welche mechanische Aufhängung (10) dazu ausgebildet ist, eine Sensorelektrode (3a, 3b, 3c) des entsprechenden Sensorelements (3) abzustützen; und
   > in die mechanische Aufhängung (10) eines der drucksensitive Messelemente (p2) aufgenommen ist.

9. Differentielles Spannungsmesssystem (100) nach einem der vorhergehenden Ansprüche, ferner mit einer Ausgabeeinheit (36), die dazu eingerichtet ist, die Atmungsinformation (A, $A_{lok}$) und wenigstens eines der bioelektrischen Signale (S1, S2) auszugeben, wobei die ausgegebenen bioelektrischen Signale (S1, S2) wenigstens einen Herzschlag des Patienten (P) indizieren.

10. Differentielles Spannungsmesssystem (100) nach einem der vorhergehenden Ansprüche, bei dem die Recheneinheit (35) ein Kalman-Filter umfasst, das dazu ausgebildet ist, basierend auf den in die Recheneinheit (35) eingegebenen Signalen (S1, S2, S3, S4, SN) die Atmungsinformation (A, $A_{lok}$) zu ermitteln und bereitzustellen.

11. Differentielles Spannungsmesssystem (100) nach einem der vorhergehenden Ansprüche, bei dem die Recheneinheit (35) dazu ausgebildet ist, eine trainierte Funktion (TF) auf die in die Recheneinheit (35) eingegebenen Signale (S1, S2, S3, S4, SN) anzuwenden, welche trainierte Funktion (TF) dazu ausgebildet ist, basierend auf den in die Recheneinheit (35) eingegebenen Signalen (S1, S2, S3, S4, SN) die Atmungsinformation (A, $A_{lok}$) zu ermitteln und bereitzustellen.

12. Verwendung des differentiellen Spannungsmesssystems (100) nach einem der vorhergehenden Ansprüche zur Synchronisation einer medizinischen Bildgebungsanlage (B) mit der Atmungsaktivität des Patienten (P).

13. Computer-implementiertes Verfahren zur Bereitstellung einer eine Atmung eines Patienten (P) indizierenden Atmungsinformation (A, $A_{lok}$) mit den Schritten:

    > Erfassen (S30) einer Anzahl auf den Patienten (P) bezogener Messignale (MS) mit wenigstens zwei kapazitiven Sensorelementen (3a, 3b, 3c);
    > Bestimmen (S40) wenigstens eines bioelektrischen Signals (S1, S2, S3, S4) aus den Messignalen (MS); und
    > Ermittlung (S70, S80) der Atmungsinformation (A, $A_{lok}$) des Patienten (P) basierend auf dem bestimmten wenigsten einen bioelektrischen Signal (S1, S2, S3, S4).

14. Verfahren nach Anspruch 13, ferner mit den Schritten:

    > Erfassen (S50) von eine Druckbelastung des Patienten (P) auf eine Unterlage (M) indizierenden Drucksignalen (DS) mit wenigstens einem drucksensitiven Messelement (p1, p2); und
    > Bestimmung (S60) wenigstens eines, insbesondere ortsaufgelösten, Druckbelastungssignals (SN) aus den Drucksignalen (DS); wobei der Schritt des Ermittelns (S70, S80) der Atmungsinformation (A, $A_{lok}$) des Patienten (P) zusätzlich basierend auf dem Druckbelastungssignal (SN) erfolgt.

**15.** Computerprogrammprodukt mit einem Computerprogramm, welches direkt in eine Speichereinrichtung eines differentiellen Spannungsmesssystems (100) ladbar ist, mit Programmabschnitten, um alle Schritte der Verfahren nach Anspruch 13 oder 14 auszuführen, wenn das Computerprogramm in dem Spannungsmesssystem (100) ausgeführt wird.

FIG 1

FIG 2

FIG 3

SVM4

MS

RFF

RS

ADF

+

+

−

S4

FIG 4

## FIG 5

## FIG 6

FIG 7

# FIG 8

S30

S40

S70 / S80

34f  34

S1  S2

35

3

MS

S3  S4

A, A$_{lok}$

SN

A

62f  62

p1 / p2

DS

SN

35

A, A$_{lok}$

SN

S50

S60

S70 / S80

FIG 9

FIG 10

Europäisches
Patentamt

European
Patent Office

Office européen
des brevets

# EUROPÄISCHER RECHERCHENBERICHT

Nummer der Anmeldung

EP 21 17 9522

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 110 464 332 A (ZHEJIANG XIANGNENG SLEEP TECH CO LTD) 19. November 2019 (2019-11-19) * das ganze Dokument * * insbesondere Absätze 18, 46 * ----- | 1-3,7,8, 10,11, 13-15 | INV. A61B5/11 A61B5/113 A61B5/00 A61B5/0245 A61B5/318 A61B5/389 |
| X | US 2016/073921 A1 (HOSKULDSSON SVEINBJORN [IS] ET AL) 17. März 2016 (2016-03-17) * Absätze [0015], [0019] - [0020], [0056], [0058], [0059]; Abbildung 4 * ----- | 1-6, 9-11,13, 15 | |
| X | DE 10 2011 076885 A1 (SIEMENS AG [DE]) 6. Dezember 2012 (2012-12-06) * Abbildungen 1,3 * * Absätze [0048], [0054] - [0058] * ----- | 1-3, 10-13,15 | |

RECHERCHIERTE
SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| Den Haag | 19. November 2021 | Albrecht, Ronald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 17 9522

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-11-2021

| Im Recherchenbericht angeführtes Patentdokument | Datum der Veröffentlichung | Mitglied(er) der Patentfamilie | Datum der Veröffentlichung |
|---|---|---|---|
| CN 110464332 A | 19-11-2019 | KEINE | |
| US 2016073921 A1 | 17-03-2016 | EP 2429390 A1<br>US 2012101357 A1<br>US 2016073921 A1<br>WO 2010131267 A1 | 21-03-2012<br>26-04-2012<br>17-03-2016<br>18-11-2010 |
| DE 102011076885 A1 | 06-12-2012 | CN 102805638 A<br>DE 102011076885 A1<br>US 2012310053 A1<br>US 2017215830 A1 | 05-12-2012<br>06-12-2012<br>06-12-2012<br>03-08-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015218298 B3
- DE 102019203627 A